# EUROPÄISCHE PATENTANMELDUNG

(11) **EP 2 719 762 A1**
(43) Veröffentlichungstag der Anmeldung: **16.04.2014**
(21) Anmeldenummer: 12007063.6
(22) Anmeldetag: 11.10.2012
(51) Int. Cl.: C12N 15/113, A61K 31/7088, A61K 31/713, A61Q 19/02

(54) **Tyrosinaseinhibitoren zur Depigmentierung oder Haarentfernung**

(71) Anmelder: Secutech International Pte. Ltd., Singapore 069443 (SG)
(72) Erfinder: Kosak, Hans, Dr., 53115 Bonn (DE); Weichert, Marcus, 13593 Berlin (DE)
(74) Vertreter: Graf von Stosch, Andreas

(57) **Zusammenfassung**

Gegenstand der vorliegenden Anmeldung sind Zusammensetzungen umfassend einen Inhibitor der Tyrosinasegenexpression, insbesondere eine Nukleinsäure, die in der Lage ist, die Genexpression des Tyrosinasegens zu inhibieren, sowie die Verwendung dieser zur Haarentfernung und/ oder Prävention des Nachwachsens von Haaren; und/oder deren Verwendung zur Depigmentierung und/oder Prävention der Repigmentierung der Haut.

SEQUENCE LISTING

<110> Secutech International pte. ltd.

<120> Tyrosinaseinhibitoren zur Depigmentierung oder Haarentfernung

<130> ST07P008EP

<160> 5

<170> PatentIn version 3.5

<210> 1
<211> 2082
<212> RNA
<213> Homo sapiens

<400> 1
aucacuguag uaguagcugg aaagagaaau cugugacucc aauuagccag uuccugcaga 60

ccuugugagg acuagaggaa gaaugcuccu ggcuguuuug uacugccugc uguggaguuu 120

ccagaccucc gcuggccauu ucccuagagc cugugucucc ucuaagaacc ugauggagaa 180

ggaaugcugu ccaccgugga gcggggacag gagucccugu ggccagcuuu caggcagagg 240

uuccugucag aauauccuuc uguccaaugc accacuuggg ccucaauuuc ccuucacagg 300

gguggaugac cgggagucgu ggccuuccgu cuuuuauaau aggaccugcc agugcucugg 360

caacuucaug ggauucaacu guggaaacug caaguuuggc uuuuggggac caaacugcac 420

agagagacga cucuugguga gaagaaacau cuucgauuug agugccccag agaaggacaa 480

auuuuuugcc uaccucacuu uagcaaagca uaccaucagc ucagacuaug ucauccccau 540

agggaccuau ggccaaauga aaaauggauc aacacccaug uuuaacgaca ucaauauuua 600

ugaccucuuu gucuggaugc auuauuaugu gucaauggau gcacugcuug ggggaucuga 660

aaucuggaga gacauugauu uugcccauga agcaccagcu uuucugccuu ggcauagacu 720

cuucuuguug cggugggaac aagaaaucca gaagcugaca ggagaugaaa acuucacuau 780

uccauauugg gacuggcggg augcagaaaa gugugacauu ugcacagaug aguacauggg 840

aggucagcac cccacaaauc cuaacuuacu cagcccagca ucauucuucu ccucuuggca 900

gauugucugu agccgauugg aggaguacaa cagccaucag ucuuuaugca auggaacgcc 960

cgagggaccu uuacggcgua auccuggaaa ccaugacaaa uccagaaccc caaggcuccc 1020

cucuucagcu gauguagaau uuugccugag uuugacccaa uaugaaucug guuccaugga 1080

uaaagcugcc aauuucagcu uuagaaauac acuggaagga uuugcuaguc cacuuacugg 1140

gauagcggau gccucucaaa gcagcaugca caaugccuug cacaucuaua ugaauggaac 1200

aaugucccag guacagggau cugccaacga uccuaucuuc cuucuucacc augcauuugu 1260

ugacaguauu uuugagcagu ggcuccgaag gcaccguccu cuucaagaag uuuauccaga 1320

agccaaugca cccauuggac auaaccggga auccuacaug guuccuuuua uaccacugua 1380

cagaaauggu gauuucuuua uuucauccaa agaucugggc uaugacuaua gcuaucuaca 1440

agauucagac ccagacucuu uucaagacua cauuaagucc uauuuggaac aagcgagucg 1500

gaucugguca uggcuccuug gggcggcgau gguaggggcc guccucacug cccugcuggc 1560

agggcuugug agcuugcugu gucgucacaa gagaaagcag cuuccugaag aaaagcagcc 1620

acuccucaug gagaaagagg auuaccacag cuuguaucag agccauuuau aaaaggcuua 1680

ggcaauagag uagggccaaa aagccugacc ucacucuaac ucaaaguaau guccagguuc 1740

ccagagaaua ucugcuggua uuuuucugua aagaccauuu gcaaaauugu aaccuaauac 1800

aaaguguagc cuucuuccaa cucagguaga acacaccugu cuuugucuug cuguuuucac 1860

ucagcccuuu uaacauuuuc cccuaagccc auaugucuaa ggaaaggaug cuauuuggua 1920

augaggaacu guuauuugua ugugaauuaa agugcucuua uuuuaaaaaa uugaaauaau 1980

uuugauuuuu gccuucugau uauuuaaaga ucuauauaug uuuuauuggc cccuucuuua 2040

uuuuaauaaa acagugagaa aucuaaaaaa aaaaaaaaaa aa 2082

<210> 2
<211> 1590
<212> RNA
<213> Homo sapiens

<400> 2
augcuccugg cuguuuugua cugccugcug uggaguuucc agaccuccgc uggccauuuc 60

ccuagagccu gugucuccuc uaagaaccug auggagaagg aaugcugucc accguggagc 120

ggggacagga gucccugugg ccagcuuuca ggcagagguu ccugucagaa uauccuucug 180

uccaaugcac cacuugggcc ucaauuuccc uucacagggg uggaugaccg ggagucgugg 240

ccuuccgucu uuuauaauag gaccugccag ugcucuggca acuucauggg auucaacugu 300

ggaaacugca aguuuggcuu uuggggacca aacugcacag agagacgacu cuuggugaga 360

agaaacaucu ucgauuugag ugccccagag aaggacaaau uuuuugccua ccucacuuua 420

gcaaagcaua ccaucagcuc agacuauguc auccccauag ggaccuaugg ccaaaugaaa 480

aauggaucaa cacccauguu uaacgacauc aauauuuaug accucuuugu cuggaugcau 540

uauuaugugu caauggaugc acugcuuggg ggaucugaaa ucuggagaga cauugauuuu 600

gcccaugaag caccagcuuu ucugccuugg cauagacucu ucuuguugcg gugggaacaa 660

gaaauccaga agcugacagg agaugaaaac uucacuauuc cauauuggga cuggcgggau 720

gcagaaaagu gugacauuug cacagaugag uacaugggag gucagcaccc cacaaauccu 780

aacuuacuca gcccagcauc auucuucucc ucuuggcaga uugucuguag ccgauuggag 840

gaguacaaca gccaucaguc uuuaugcaau ggaacgcccg agggaccuuu acggcguaau 900

ccuggaaacc augacaaauc cagaacccca aggcuccccu cuucagcuga uguagaauuu 960

ugccugaguu ugacccaaua ugaaucuggu uccauggaua aagcugccaa uuucagcuuu 1020

agaaauacac uggaaggauu ugcuagucca cuuacuggga uagcggaugc cucucaaagc 1080

agcaugcaca augccuugca caucuauaug aauggaacaa ugucccaggu acagggaucu 1140

gccaacgauc cuaucuuccu ucuucaccau gcauuuguug acaguauuuu ugagcagugg 1200

cuccgaaggc accguccucu ucaagaaguu uauccagaag ccaaugcacc cauuggacau 1260

aaccgggaau ccuacauggu uccuuuuaua ccacuguaca gaaaugguga uuucuuuauu 1320

ucauccaaag aucugggcua ugacuauagc uaucuacaag auucagaccc agacucuuuu 1380

caagacuaca uuaaguccua uuuggaacaa gcgagucgga ucuggucaug gcuccuuggg 1440

gcggcgaugg uaggggccgu ccucacugcc cugcuggcag ggcuugugag cuugcugugu 1500

cgucacaaga gaaagcagcu uccugaagaa aagcagccac uccucaugga gaaagaggau 1560

uaccacagcu uguaucagag ccauuuauaa 1590

<210> 3
<211> 19
<212> DNA
<213> Artificial

<220>
<223> Artificial Oligonucleotide

<400> 3
gtgatggtat acgtcctag 19

<210> 4
<211> 22
<212> DNA
<213> Artificial Sequence

<220>
<223> Artificial Oligonucleotide

<220>
<221> misc_feature
<222> (1)..(19)
<223> RNA

<220>
<221> misc_feature
<222> (20)..(21)
<223> Desoxythymidine

<400> 4
cuaucugaau cagauucuad tt 22

<210> 5
<211> 21
<212> DNA
<213> Artificial

<220>
<223> Artificial Oligonucleotide

<220>

<221>  misc_feature
<222>  (1)..(19)
<223>  RNA

<220>
<221>  misc_feature
<222>  (20)..(21)
<223>  Desoxythymidine

<400>  5
gcugacccug aaguucauct t                                                21

## Beschreibung

Gegenstand der vorliegenden Anmeldung sind Zusammensetzungen umfassend einen Inhibitor der Tyrosinasegenexpression, insbesondere eine Nukleinsäure, die in der Lage ist, die Genexpression des Tyrosinasegens zu inhibieren, sowie die Verwendung dieser zur Haarentfernung und/oder Prävention des Nachwachsens von Haaren; und/oder deren Verwendung zur Depigmentierung und/oder Prävention der Repigmentierung der Haut.

### Stand der Technik

Haarwachstum ist ein charakteristisches Merkmal der Haut von Menschen und Tieren, insbesondere Säugetieren. Das Haarkleid nimmt dabei zahlreiche Funktionen wahr und dient z.B. dem Schutz vor UV-Strahlung, der Wärmedämmung, dem Feuchtigkeitsschutz, und der Feuchtigkeitsregulierung. Aus hygienischen Gründen wie kosmetischen Gründen kann jedoch Haarwachstum unerwünscht sein, beispielsweise in den Achselhöhlen, an Fingern, der Handoberfläche, Armen, Beinen, dem Fußrücken oder in der Schamgegend oder auf Brust, Bauch und/oder Rücken und/oder im Gesichtsbereich. Ebenso gibt es Erkrankungsformen wie Hypertrichose (Überbehaarung) und Hirsutismus (männlicher Behaarungstyp bei Frauen), bei denen das Wachstum von Haaren dereguliert ist.

Die Pigmentierung der Haut dient, ebenso wie das Haarkleid, auch dem Schutz vor UV-Strahlung. Aber auch in diesem Fall kann aus kosmetischen oder gesundheitlichen Gründen (z.B. bei Hyperpigmentierung) die Pigmentierung unerwünscht sein.

Sowohl für die Haarentfernung als auch die Depigmentierung (z.B. Bleichen der Haut) sind zahlreiche Verfahren, Zusammensetzungen und Vorrichtungen im Stand der Technik bekannt. Trotzdem besteht nach wie vor ein Bedarf an neuen Methoden zur Haarentfernung und/oder Prävention des Nachwachsens von Haaren; und/oder Depigmentierung und/oder Prävention der Repigmentierung der Haut.

### Zusammenfassung der Erfindung

Die Erfinder der vorliegenden Erfindung haben überraschenderweise festgestellt, dass ein Inhibitor der Tyrosinasegenexpression (beispielhaft gezeigt mit Hilfe einer siRNA) zur Haarentfernung und/oder Prävention des Nachwachsens von Haaren; und/oder zur Depigmentierung und/oder Prävention der Repigmentierung der Haut verwendet werden kann.

Die Erfindung betrifft daher in einem ersten Aspekt eine Zusammensetzung umfassend einen Inhibitor der Tyrosinasegenexpression, wobei der Inhibitor der Tyrosinasegenexpression vorzugsweise i) eine Nukleinsäure ist, die in der Lage ist, die Genexpression des Tyrosinasegens (Tyr Gen) zu inhibieren, und/oder ii) ein Inhibitor des Microphtalmia-assoziierten Transkriptionsfaktors (MITF) ist, der in der Lage ist, die MITF vermittelte Transkription des Tyr-Gens zu inhibieren. In besonders bevorzugten Ausführungsformen dieser Erfindung ist der erfindungsgemäße Inhibitor der Tyrosinasegenexpression eine Nukleinsäure, insbesondere eine siRNA, die in der Zusammensetzung in Form von Komplexen mit mindestens einem organischen Kation in mindestens einen organischen Lösungsmittel gelöst vorliegt.

In einem weiteren Aspekt betrifft die vorliegende Erfindung die Verwendung einer erfindungsgemäßen Zusammensetzung bei einem Tier oder Menschen zur:
i) Haarentfernung und/oder Prävention des Nachwachsens von Haaren; und/oder
ii) Depigmentierung und/oder Prävention der Repigmentierung der Haut.

Diese Verwendung kann nicht-therapeutischen, nicht-chirurgischen Zwecken dienen, z.B. rein kosmetischen Zwecken ohne therapeutischen Grund oder chirurgischen Eingriff, sie kann aber auch therapeutischen Zwecken dienen und/oder begleitend zu einem chirurgischen Eingriff angewendet werden. Beispielsweise können die erfindungsgemäßen Zusammensetzungen zur Behandlung von Hypertrichose, Hirsutismus, und/oder Hyperpgimentierung verwendet werden.

Dementsprechend betrifft die vorliegende Erfindung auch Verfahren zur
i) Haarentfernung und/oder Prävention des Nachwachsens von Haaren; und/oder
ii) Depigmentierung und/oder Prävention der Repigmentierung der Haut,
   bei einem Tier oder Menschen.

Mit einem derartigen Verfahren konnten die Erfinder der vorliegenden Erfindung eine lang anhaltende Wirkung bei einfacher Art der dermalen Applikation beobachten.

### Detaillierte Beschreibung der Erfindung

Die Erfinder der vorliegenden Erfindung konnten am Beispiel einer siRNA gegen das Tyrosinasegen in Mäusen zeigen, dass durch gezielte Inhibition der Tyrosinasegenexpression in der Haut Haarausfall und Depigmentierung bewirkt werden kann.

Erfindungsgemäß wird unter der "Tyrosinasegenexpression" die Expression des Tyrosinasegens (Tyr) in einem Menschen oder Tier (insbesondere einem Säugetier) verstanden. Darunter fallen alle dem Fachmann bekannten Stufen des Expressionsprozesses, beispielsweise die Transkription des Tyr-Genlokus, die anschließenden RNA-Prozessierung (z.B. Spleißen), der Translationsprozess, als auch posttranslationale Prozessierungen des translatierten Proteinprodukts zum Endprodukt, der enzymatisch aktiven Tyrosinase. Ein Inhibitor der Tyrosinasegenexpression liegt vor, wenn mindestens eine dieser Stufen des Expressionsprozesses inhibiert wird.

Erfindungsgemäß umfasst die erfindungsgemäße Zusammensetzung einen Inhibitor der Tyrosinasegenexpression. Dieser Inhibitor ist eine Verbindung (oder eine Kombination von Verbindungen), der die Tyrosinasegenexpression stört. Die Störung der Genexpression kann wie erwähnt dabei auf einer beliebigen Stufe des Expressionsprozesses erfolgen. Wie hierein verwendet inhibiert ein Inhibitor der Tyrosinasegenexpression die Expression der Tyrosinase in vitro (und vorzugsweise auch in vivo) auf der entsprechenden Stufe um mindestens 20%, noch bevorzugter um mindestens 30%, noch bevorzugter um mindestens 40%, noch bevorzugter um mindestens 50%, noch bevorzugter um mindestens 60%, noch bevorzugter um mindestens 70%, noch bevorzugter um mindestens 80%, noch bevorzugter um mindestens 90%, am bevorzugtesten um mindestens 95% im Vergleich zur Genexpressionsrate (der entsprechenden Stufe) in Abwesenheit des Inhibitors. Zahlreiche Verfahren zur Bestimmung der Genexpressionsrate sind dem Fachmann aus dem Stand der Technik bekannt. Verringerungen des Transkriptionsniveaus des Tyr-Gens können beispielsweise mit Hilfe quantitativer PCR-Verfahren nachgewiesen werden, Verringerungen auf der Translationsebene können beispielweise mit quantitativen Proteinnachweismethoden wie beispielsweise quantitativem Western Blot, quantitativer Fluoreszenzspektroskopie, Massenspektrometrie etc. nachgewiesen werden. Als entsprechendes Zelltestsystem kommen Tyrosinase exprimierende Zellen in Frage, z.B. Melanocyten oder auch Hautgewebeschnitte.

Unter dem erfindungsgemäßen Inhibitor ist kein ubiquitärer Inhibitor der Genexpression zu verstehen, d.h. kein Inhibitor der generell die Genexpression in einer Zelle inhibiert (und dadurch unter anderem auch die Expression des Tyrosinasegens inhibiert), sondern vorzugsweise ein (mehr oder weniger) spezifischer Inhibitor der Tyrosinasegenexpression, Zudem handelt es sich bei dem erfindungsgemäßen Inhibitor vorzugsweise um einen reversiblen Inhibitor, d.h. in Gegenwart des Inhibitors ist die Tyrosinasegenexpression inhibiert, nach Entfernung des Inhibitors nimmt die Tyrosinasegenexpression jedoch wieder zu.

Ein Beispiel für einen Inhibitor ist beispielsweise eine Inhibitor des Microphtalmia-assoziierten Transkriptionsfaktors (MITF). MITF ist ein für die Transkription des Tyr-Gens verantwortlicher Transkriptionsfaktor. Jeder Inhibitor von MITF inhibiert daher zumindest mittelbar die Expression des Tyrosinasegens, da der entsprechend benötigte Transkriptionsfaktor nicht oder nur in verringertem Maße vorliegt bzw. aktiv ist. Ein Inhibitor von MITF kann beispielsweise auf enzymatischer Ebene wirken, z.B. als allosterischer Inhibitor, ein kompetitiver Inhibitor hinsichtlich der Bindungsstelle am Tyrosinasegen sein, oder ein alternatives Substrat darstellen, d.h. MITF bindet an dieses Substrat und kann im selben Umfang nicht an das eigentliche Substrat binden. Es kann sich auch um eine Nukleinsäure, z.B. siRNA handeln, die die Expression von MITF selbst verringert (z.B. anti-MITF-Antisense-RNA, anti-MITF-siRNA oder anti-MITF-micro RNA). Dem Fachmann sind zahlreiche technische Möglichkeiten bekannt um die Expression von MITF, und damit die Expression der Tyrosinase, zu inhibieren.

Vorzugsweise ist der Inhibitor in der erfindungsgemäßen Zusammensetzung eine Nukleinsäure, die in der Lage ist, die Genexpression des Tyrosinase Gens (Tyr Gen) zu inhibieren. Unter dem Begriff "Nukleinsäure" ist erfindungsgemäß eine Nukleinsäure zu verstehen, deren Nukleoside durch Phosphodiesterbindungen miteinander verknüpft sind, wobei die an den Phosphodiesterbindungen beteiligten Phosphatreste negativ geladen sind, wie es bei natürlich vorkommender DNA oder RNA der Fall ist. Mit anderen Worten handelt es sich bei den geladenen Nukleinsäuren um polyanionische Säuren (Polyelektrolyte), in denen die anionischen Gruppen durch die negativ geladenen Sauerstoffreste der Phosphatgruppen, die die Phosphordiesterbindungen bilden, gebildet werden. Grundsätzlich kann es sich bei den die negativen Ladungen tragenden Resten der Nukleinsäuren auch um die negativen Reste von Phosphorthioaten oder Phosphordithioaten oder auch um andere negativ geladene Gruppen von Nukleinsäuren handeln. Die Nukleotide der Nukleinsäure können natürlich vorkommende Nukleotide sein, die Nukleinsäure kann aber auch modifizierte Nukleotide umfassen, beispielsweise 2'-O-Methyl modifizierte Nukleotide, Nukleotide mit einer 5'-Phosphorothioatgruppe, oder terminale Nukleotide die mit einem Cholesterinderivat verknüpft sind. 2' modifizierte Nukleotide können den Vorteil haben, dass bestimmte Immunreaktionen unterdrückt werden. Als modifizierte Nukleotide können beispielsweise 2'-Deoxy-2'-fluor-modifizierte Nukleotide, 2'-Deoxy-modifizierte Nukleotide, LNA-Nukleotide (locked nucleic acid; es liegt eine Methylenbrücke zwischen dem 2'-Sauerstoff der Ribose und dem 4' Kohlenstoff der Ribose vor), abasische Nukleotide, 2'-amino-modifizierte Nukleotide, 2'-Alkyl modifizierte Nukleotide, Morpholinonukleotide, Phosphoramidate, Nukleotide mit nichtnatürlichen Basen etc. verwendet werden. Bevorzugte modifizierte Nukleotide, insbesondere bei doppelsträngigen RNA-Molekülen wie bei einer siRNA sind 2'-O-Methyl modifizierte Nukleotide und Nukleotide mit einer 5'-Phosphorothioatgruppe. Dem Fachmann ist bekannt, wie er Nukleinsäuren mit chemisch modifizierte Nukleotiden herstellen kann (siehe z.B. Publikationsreihe "Current protocols in nucleic acid chemistry", John Wiley & Sons, Inc., New York, NY, USA).

Die Nukleinsäure kann als DNA, als RNA, als DNA/RNA-Hybrid oder als DNA/RNA-Mischsequenz (in einem Strang existieren Ribose- und Desoxyribosereste) vorliegen. Die Nukleinsäure kann als Einzelstrang vorliegen, aber auch als Doppelstrang. Der Begriff "Doppelstrang", wie hierin verwendet, soll nicht das Vorliegen von einzelsträngigen Bereichen, z.B. Überhängen am 3' Ende oder Hairpinloops, ausschließen. Eine doppelsträngige Nukleinsäure kann bspw. in Form zweier separater Einzelstränge oder als Haarnadelschleifenstruktur vorliegen. Bei den Nukleinsäuren kann es sich beispielsweise um DNA, cDNA, Antisense-DNA, RNA, Antisense RNA, siRNA, Decoys, und/oder miRNA handeln.

In bevorzugten Ausführungsformen der Erfindung handelt es sich bei der Nukleinsäure um ein Oligonukleotid, d.h. um einzelsträngige oder doppelsträngige DNA oder RNA (bzw. DNA/RNA-Hybrid oder DNA/RNA-Mischsequenzen) mit einer Kettenlänge von etwa 5 bis etwa 150 Nukleotiden, bevorzugt von etwa 7 bis etwa 100 Nukleotiden, bevorzugt etwa 10 bis etwa 80 Nukleotiden, besonders bevorzugt etwa 15 bis etwa 60 Nukleotiden.

In solchen Ausführungsformen der Erfindung kann die Nukleinsäure beispielsweise eine RNA sein, insbesondere eine anti-Tyr-Antisense-RNA, anti-Tyr-siRNA oder anti-Tyr-micro RNA. Die Erzeugung von Antisense-RNA, siRNAs und micro RNAs ist im Stand der Technik wohl bekannt und der Fachmann ist in der Lage unter Zuhilfenahme der öffentlich verfügbaren Sequenzinformationen, z.B. zum Tyrosinasegen, und zugehöriger kodierender Sequenz entsprechende Moleküle herzustellen oder von Dritten synthetisieren zu lassen. Sequenzen der humanen Tyrosinase mRNA (SEQ ID NO: 1) und der entsprechenden kodierenden Sequenz (SEQ ID NO: 2) sind beispielhaft hierin offenbart.

Die Nukleinsäure kann, insbesondere wenn es sich um eine anti-Tyr-Antisense-RNA oder anti-Tyr-siRNA handelt, einen Sequenzabschnitt umfassen, der eine Sequenzidentität von mindestens 70%, vorzugsweise von mindestens 80%, noch bevorzugter von mindestens 90%, und am bevorzugtesten von 100% mit einem Sequenzabschnitt der kodierenden Sequenz des Tyrosinasgens, z.B. der kodierenden Sequenz der humanen Tyrosinase mRNA (SEQ ID NO: 2) oder, noch bevorzugter, mit einer dazu komplementären Sequenz umfassen. Vorzugsweise ist dieser Sequenzabschnitt weniger als etwa 30 Nukleotide lang. Besonders bevorzugt weist der Sequenzabschnitt entsprechender Sequenzidentität eine Länge im Bereich von etwa 16 bis 30 Nukleotiden, noch bevorzugter von etwa 18 bis 28 Nukleotiden, noch bevorzugter eine Länge von 19, 20, 21, 22, 23 oder 24 Nukleotiden auf. Am bevorzugtesten liegt ein Abschnitt mit einer Länge von 19 bis 23 Nukleotiden entsprechender Sequenzidentität vor. Die Nukleinsäure kann, beispielsweise im Fall einer siRNA, insbesondere einer anti-Tyr-siRNA, als Doppelstrang vorliegen und, neben dem Bereich der Sequenzidentität am 3'Ende eines oder beider Stränge (dann jeweils unabhängig voneinander) einen einzelsträngigen Überhang von 1 bis 5 Nukleotiden aufweisen, vorzugsweise einen Überhang von 1 bis 2 Nukleotiden. Besonders bevorzugt ist es, wenn die überhängenden Nukleotide dT (Deoxythymidin) Nukleotide sind. Die Nukleinsäure, insbesondere die siRNA, kann, wenn sie als Doppelstrang vorliegt, Basenfehlpaarungen umfassen, aber vorzugsweise nicht mehr als 4, noch bevorzugter nicht mehr als 3, noch bevorzugter nicht mehr als 2.

Der Grad an Sequenzidentität zwischen zwei Sequenzen lässt sich in der Regel wie erfolgt ermitteln: Zwei Sequenzen werden so nebeneinandergelegt, dass die maximale Anzahl an Übereinstimmungen erzielt wird. Dazu können in einer oder, falls notwendig, in beiden Sequenzen Lückenpositionen (Gaps) eingefügt werden, um die Anzahl an Übereinstimmung zu erhöhen. Der Grad an Sequenzidentität kann dann über die gesamte Länge der Sequenzen samt Lückenpositionen erfolgen. Bei Sequenzen im wesentlichen gleicher Länge wird dazu vorzugsweise ein globaler Vergleich (globales Alignment) durchgeführt, bei Sequenzen ungleicher Länge ein lokaler Vergleich (lokales Alignment). Im diesem Kontext weist beispielsweise eine Sequenz, die 95% Sequenzidentität mit einer anderen RNA-Sequenz aufweisen soll, im Vergleich zu dieser fünf Sequenzabweichung pro 100 Sequenzpositionen auf. Mit anderen Worten, fünf Sequenzpositionen können zusätzlich eingefügt werden, anders substituiert sein und/oder fehlen.

In besonders bevorzugten Ausführungsformen der vorliegenden Erfindung ist der Inhibitor eine doppelsträngige siRNA, die die in der Lage ist, die Genexpression des humanen Tyrosinase Gens (Tyr Gen) zu inhibieren, und die folgendes umfasst:
i) einen Sequenzabschnitt einer Länge von 19 bis 23 Nukleotiden, der mindestens 70% Sequenzidentität mit einem Sequenzabschnitt der kodierenden Sequenz der humanen Tyrosinase mRNA (SEQ ID NO: 2) oder einer dazu komplementären Sequenz aufweist, und
ii) am 3'Ende eines oder beider Stränge einen einzelsträngigen Überhang von 1 bis 2 Nukleotiden aufweist, die vorzugsweise dT (Deoxythymidin) Nukleotide sind.

Für die Maus sind doppelsträngige siRNA Moleküle mit einer Sequenz gemäß SEQ ID NO: 4 besonders bevorzugt.

Neben den oben diskutierten Oligonukleotiden kann die Nukleinsäure aber auch ein Polynukleotide mit mehr als 150 Nukleotiden sein. In einer besonderen Ausführungsform der Erfindung handelt es sich bei der Nukleinsäure um einen Vektor oder um ein Plasmid mit einer Größe von mehr als 1 kb (1000 Nukleotide). Die Größe des Vektors oder des Plasmids wird in der Regel mehr als 2 kb, 3 kb, 4 kb, 5 kb, 6 kb, 7 kb, 8 kb, 9 kb, 10 kb, 11 kb, 12 kb, 13 kb, 14 kb, 15 kb oder sogar mehr als 20 kb betragen. Es kann sich bei der Nukleinsäure z.B. um einen Expressionsvektor handeln, der für die Expression mindestens eines Gens in die Zellen eingebracht wird. Beispiele für Expressionsvektoren, die sich eignen, umfassen virale und nicht-virale Vektoren. Virale Vektoren leiten sich von verschiedenen Viren ab, z.B. von Retroviren, Herpesviren, Adenoviren oder von Adeno-assoziierten Viren (AAV), siehe Lundstrom, Trends Biotechnol. (2003), 21 (3):117-22. Vorzugsweise handelt es sich bei den viralen Vektoren um solche, die nicht mehr in der Lage sind, sich in den damit transfizierten Zellen zu replizieren. Neben den viralen Vektoren können auch nicht-virale Vektoren, z.B. eukaryontische Expressionsvektoren, wie vorliegend beschrieben, verwendet werden. Die Vektoren oder Plasmide umfassen vorzugsweise einen Promotor, der in Tyrosinase exprimierenden Zellen aktiv ist. Derartige Vektoren können dann beispielsweise für eine anti-Tyr-Antisense-RNA, anti-Tyr-siRNA, anti-Tyr-micro RNA, oder anderweitige Inhibitoren der Tyrosinasegenexpression kodieren.

Wie hierin gezeigt, kann die erfindungsgemäße Zusammensetzung, wenn der Inhibitor eine Nukleinsäure ist, mindestens ein organisches Lösungsmittel umfassen. Das organische Lösungsmittel enthält darin gelöst die mit einem oder mehreren organischen Kationen komplexierte Nukleinsäure. Eine Nukleinsäure wird im Sinne der Erfindung als gelöst erachtet, wenn sie sich durch eine 5-minütige Zentrifugation bei 15000 x g nicht abzentrifugieren lässt. Üblicherweise lösen sich Nukleinsäuren auf Grund ihrer Ladung gut in Wasser, nicht jedoch in mit Wasser nicht mischbaren organischen Flüssigkeiten, wie Kohlenwasserstoffen oder anderen organischen Lösungsmitteln, wie Chloroform, Ölen etc. Die Nukleinsäure ist in diesen Ausführungsformen in der Regel aber derart komplexiert, dass die negativen Ladungen der Nukleinsäure durch positive Ladungen der organischen Kationen im Wesentlichen vollständig kompensiert werden, vorzugsweise durch Ausbildung von Ionenpaaren. Dies erlaubt die Lösung in organischen Lösungsmitteln. Im Wesentlichen vollständig kompensiert bedeutet hier insbesondere, dass das Verhältnis der Anzahl an positiven Ladungen der Kationen zu negativen Ladungen der Nukleinsäure vorzugsweise größer oder gleich 0,5 ist, vorzugsweise größer oder gleich 0,6 ist, vorzugsweise größer oder gleich 0,7 ist, vorzugsweise größer oder gleich 0,8 ist, vorzugsweise größer oder gleich 0,9 ist, vorzugsweise größer oder gleich 0,91 ist, vorzugsweise größer oder gleich 0,92 ist, vorzugsweise größer oder gleich 0,93 ist, vorzugsweise größer oder gleich 0,94 ist, vorzugsweise größer oder gleich 0,95 ist, vorzugsweise größer oder gleich 0,96 ist, vorzugsweise größer oder gleich 0,97 ist, vorzugsweise größer oder gleich 0,98 ist, vorzugsweise größer oder gleich 0,985 ist, vorzugsweise größer oder gleich 0,99 ist, vorzugsweise größer oder gleich 0,995 ist, oder sogar gleich 1 ist. Das Verhältnis der Anzahl an positiven Ladungen der Kationen zu negativen Ladungen der Nukleinsäure kann beispielsweise im Bereich 0,5 - 2 liegen, z.B. im Bereich 0,6 - 1,8 liegen, im Bereich 0,7 - 1,3, im Bereich 0,8 - 1,2 liegen, im Bereich 0,9 - 1,1 liegen, im Bereich 0,91 - 1,09 liegen, im Bereich 0,92 - 1,08 liegen, im Bereich 0,93 - 1,07 liegen , im Bereich 0,94 - 1,06 liegen, im Bereich 0,95 - 1,05 liegen, im Bereich 0,96 - 1,04 liegen, im Bereich 0,96 - 1,04 liegen, im Bereich 0,97 - 1,03 liegen, im Bereich 0,98 - 1,02 liegen, im Bereich 0,99 - 1,01 liegen, im Bereich 0,995 - 1,005 liegen, oder sogar gleich 1 sein. Letzterer Wert wird insbesondere dann erzielt, wenn bei der Herstellung (siehe später) Kationen bei der Fällung der Nukleinsäure im Überschuss vorliegen und das Präzipitat aus organischen Kation und Nukleinsäure mit Wasser gewaschen wird (siehe später). Die Zusammensetzung wird dann im Wesentlichen keine freien, nicht komplexierten organischen Kationen umfassen.

Es wurde festgestellt, dass es durch diese Form der Komplexierung möglich ist, Nukleinsäuren, die ansonsten in organischen Lösungsmitteln nicht löslich sind, in hohen Mengen in solche Lösungsmittel einzubringen. Nukleinsäuren, wie z.B. DNA oder RNA, sind unter physiologischen Bedingungen anionische Polymere, die über eine hohe Anzahl von negativ geladenen Phosphat-Gruppen verfügen. Aufgrund der Ladung der Polymere sind diese in wässriger Lösung gut löslich, in organischen Lösungsmitteln dagegen eigentlich nicht.

Die hierin beschriebenen komplexierten Nukleinsäuren lassen sich jedoch in organischen Lösungsmitteln lösen. Für diese konnte gezeigt werden, dass sie sich auch in wirksamer Weise in Zellen und/oder Gewebe z.B. eines Säugetiers einbringen lassen.

"Mindestens ein organisches Kation" bedeutet, dass mindestens ein Typ an organischem Kation vorliegt (z.B. Cetyltrimethylammoniumionen, Dimethyldioctadecylammoniumionen, oder Benzalkoniumionen). Es können aber auch Kationengemische von zwei oder mehr, drei oder mehr, vier oder mehr, fünf oder mehr, etc. unterschiedlichen organischen Kationen vorliegen (z.B. Cetyltrimethylammoniumionen neben Dimethyldioctadecylammoniumionen). Üblicherweise ist das mindestens eine organische Kation einfach positiv geladen.

"Mindestens ein organisches Lösungsmittel" bedeutet, dass mindestens ein organisches Lösungsmittel vorliegt. Es können aber auch Lösungsmittelgemische von zwei oder mehr, drei oder mehr, vier oder mehr, fünf oder mehr, etc. unterschiedlichen organischen Lösungsmitteln vorliegen. Die Nukleinsäuren sind dann entsprechend in diesen Lösungsmittelgemischen gelöst. Der Begriff soll auch nicht ausschließen, dass ein Gemisch (mindestens) eines organischen Lösungsmittels mit einer wässrigen Flüssigkeit vorliegt. Organische Flüssigkeiten wie Alkohole, in denen die hier beschriebenen Komplexe löslich sind, lassen sich mit Wasser beliebig mischen und bilden einen homogene Phase. Daher können die Komplexe über die Lösung in derartigen organischen Lösungsmitteln auch in wässrige Medien eingebracht werden. Die Nukleinsäuren sind dann entsprechend in diesem Lösungsmittelgemisch aus (mindestens) einem organischen Lösungsmittel und Wasser gelöst. Mit anderen Worten: erfindungsgemäß liegen die Komplexe dann in der Zusammensetzung in einer homogenen Phase gelöst vor. Diese homogene Phase wiederum umfasst mindestens ein organisches Lösungsmittel.

Falls der Inhibitor in der Tyrosinasegenexpression eine Nukleinsäure ist, umfassen die erfindungsgemäßen Zusammensetzungen mindestens eine Nukleinsäure, die in der Lage ist, die Genexpression des Tyrosinase Gens (Tyr Gen) zu inhibieren. "Mindestens eine Nukleinsäure" bedeutet, dass mindestens eine Nukleinsäure vorliegt. Es können aber auch Nukleinsäuregemische von zwei oder mehr, drei oder mehr, vier oder mehr, fünf oder mehr, etc. unterschiedlichen Nukleinsäuren vorliegen, die ggf. alle einzeln für sich oder zusammen in der Lage sind, die Genexpression des Tyrosinase Gens (Tyr Gen) zu inhibieren. Bei derartigen Nukleinsäuregemischen kann die Art der Nukleinsäure unterschiedlich sein, z.B. eine Mischung von DNA und RNA, es kann aber auch lediglich ein Unterschied in der Sequenz vorliegen.

Die mindestens eine Nukleinsäure löst sich in den organischen Lösungsmitteln aufgrund der Ladungskompensation in den gebildeten Komplexen. Die Lösung der Nukleinsäure benötigt daher nicht die Bildung von Micellen, Liposomen oder ähnlichen nanopartikulären Strukturen. Üblicherweise liegen daher Komplexe aus mindestens einer Nukleinsäure und mindestens einem organischen Kation gelöst vor, wobei die Komplexe sich nicht im Inneren einer Micelle, eines Liposoms oder ähnlicher nanopartikulärer Strukturen befinden oder die Nukleinsäuren an die äußere Oberfläche einer Micelle, eines Liposoms oder einer ähnlichen nanopartikulären Struktur adsorbiert sind. Üblicherweise, ohne jedoch darauf beschränkt zu sein, enthalten die hier verwendeten Zusammensetzungen daher keine Micellen, Liposomen oder ähnliche nanopartikuläre Strukturen.

Die erfindungsgemäße Zusammensetzung kann beispielsweise bereitgestellt werden, indem Komplexe aus mindestens einer Nukleinsäure, die in der Lage ist, die Genexpression des Tyrosinase Gens (Tyr Gen) zu inhibieren (z.B. eine anti-Tyr siRNA), und mindestens einem organischen Kation in einem organischen Lösungsmittel oder einem Gemisch von organischen Lösungsmitteln gelöst werden und gegebenenfalls dann mit einer wässrigen Flüssigkeit gemischt werden. Zur Gewinnung der Komplexe kann zunächst die mindestens eine Nukleinsäure in einer wässrigen Flüssigkeit, insbesondere Wasser (z.B. demineralisiertes Wasser) gelöst werden, anschließend durch Zugabe von mindestens einem organischem Kation, das mit der Nukleinsäure einen in der wässrigen Lösung unlöslichen Komplex bildet, präzipitiert werden, und das Präzipitat ggf. gewaschen und, z.B. durch Trocknung, isoliert werden. Das Präzipitat ist vorzugsweise frei von jeglichen wässrigen Rückständen und unlöslich in Wasser.

In Ausführungsformen der Erfindung mit einer Nukleinsäure als Inhibitor, die in der Lage ist, die Genexpression des Tyrosinase Gens (Tyr Gen) zu inhibieren, kann die erfindungsgemäße Zusammensetzung daher zum Beispiel mit einem Verfahren hergestellt werden, dass folgende Schritte umfasst:
i) Bereitstellen einer Lösung mit mindestens einer in einer wässrigen ersten Flüssigkeit gelösten Nukleinsäure, die in der Lage ist, die Genexpression des Tyrosinase Gens (Tyr Gen) zu inhibieren, z.B. eine anti-Tyr siRNA,
ii) Präzipitieren der mindestens einen Nukleinsäure durch Zusatz mindestens eines mit der Nukleinsäure in der ersten Flüssigkeit unlösliche Komplexe bildenden organischen Kations (Komplexbildner) zu der ersten Flüssigkeit,
iii) Abtrennen der Komplexe von der ersten Flüssigkeit, z.B. durch Trocknung,
iv) Lösen der Komplexe in mindestens einem organischen Lösungsmittel (zweite Flüssigkeit)
v) optional Mischen der zweiten Flüssigkeit mit einer dritten Flüssigkeit, z.B. einem weiteren organischen Lösungsmittel wie einem Öl oder einer wässrigen Lösung etc.

Geeignete organische Kationen im Rahmen der vorliegenden Erfindung (bzw. Quellen für die Kationen) sind dabei beispielsweise kationische Detergenzien, Lipide und Ammoniumverbindungen, wie z.B. organische Ammoniumsalze. Zum Beispiel im Fall von Ammoniumverbindungen ist dann die mindestens eine Nukleinsäure in der Zusammensetzung derart komplexiert, dass die negativen Phosphatgruppen der Nukleinsäure durch Ammoniumgruppen der Ammoniumverbindung im Wesentlichen vollständig kompensiert sind. Insbesondere können als Quelle für organische Kationen quartäre Ammoniumverbindungen verwendet werden. Dazu können eine oder mehrere Ammoniumverbindungen der Formel NR₄X verwendet werden, wobei jeder Rest R jeweils unabhängig voneinander ein verzweigter oder unverzweigter Kohlenwasserstoffrest mit 1 bis 20 Kohlenstoffatomen ist, und wobei X ein Halogenid-Ion ausgewählt aus der Gruppe bestehend aus Chlorid, Bromid und Iodid ist. Bevorzugt sind Chlorid oder Bromid.

Insbesondere können einer oder mehrere, insbesondere zwei oder drei, Reste R relativ kurzkettige Reste wie C1 bis C3, wie Methyl, Ethyl und Propyl sein, während ein Rest R ein längerer Rest wie C8 oder C10 bis C20, insbesondere C10 bis C16, wie Octyl, Nonyl, Decyl, Undecyl, Dodecyl, Tridecyl, Tetradecyl, Pentadecyl, Cetyl, Heptadecyl, Icosanyl, Stearyl oder Nonadecyl ist.

In einigen Ausführungsformen der Erfindung kann das mindestens eine organische Kation eine quartäre Ammoniumverbindung sein, wobei ein, zwei oder drei Reste ein C1 Rest sind, insbesondere Methyl, und entsprechend die anderen 3, 2 oder 1 Reste organische Reste darstellen, insbesondere längerkettige Reste mit z.B. mindestens 6 Kohlenstoffatomen. So können als organische Kationen Ammoniumionen vorliegen, bei denen zwei der vier Reste der quartären Ammoniumverbindung Methylgruppen sind und die anderen beiden Reste organische Reste mit mindestens 6 Kohlenstoffatomen sind. Ein Beispiel hierfür sind Benzalkoniumionen und Dimethyldioctadecylammoniumionen. Andere Beispiele sind quartäre Ammoniumionen, bei denen drei der vier Reste Methylgruppen sind und der andere organische Rest mindestens 6 Kohlenstoffatome hat. Ein Beispiel hierfür sind Cetyltrimethylammoniumionen.

Vorzugsweise handelt es sich bei dem mindestens einen organischen Kation (bzw. der Quelle für das Kation) um ein oder mehrere quartäre Amine, wobei CTAB besonders bevorzugt ist. Andere Verbindungen, die sich erfindungsgemäß für die Komplexierung der Nukleinsäuren eignen, umfassen beispielsweise Benzethoniumchlorid, Benzalkonium, Benzalkoniumchlorid, Didecyldimethylammoniumbromid (DCAB), Dodecyltrimethylammoniumbromid (DCTAB), DOTAP, Lipofectin, Lipofectamin N-[1-(2,3-dioleyloxy)propyl]-N,N,N-trimethylammonium-chloride (DOTMA), Dimethyldioctadecyl-ammoniumbromid (DDAB), Dioleyldimethylammoniumchloride (DODAC), 2,3-Dioleoyloxy-N-[2-(spermidin carboxyamido)ethyl]-N-Ndimethyl-1-propaniniumtrifluoracetat (DOSPA), Diheptadecylamido-glycylspermidin (DHGS) und ähnliche.

Vorzugsweise wird der Komplexbildner der ersten Flüssigkeit in Schritt ii) in gelöster Form zugesetzt. Dadurch wird eine schnellere Präzipitation ermöglicht als beim Zusatz eines ungelösten Komplexbildners. Darüber hinaus kann der Komplexbildner im, vorzugsweise leichten, Überschuss zugesetzt werden. Aber auch im Unterschuss kann der Komplexbildner bereits vereinzelt zu Präzipitierung der Komplexe führen. Ziel ist es, dafür zu sorgen, dass die Nukleinsäure mit dem mindestens einen organischen Kation vorzugsweise so komplexiert wird, dass die negativen Ladungen der Nukleinsäuren durch positive Ladungen der organischen Kationen im Wesentlichen vollständig kompensiert sind, und insbesondere vorzugsweise ein Verhältnis von im Wesentlichen 1:1 zwischen den negativen Ladungen (d.h. den anionischen Gruppen) der mindestens einen Nukleinsäure und den Ladungen des mindestens einen organischen Kations vorliegt. Insbesondere organischen Kationen wie die oben erwähnten, z.B. Cetyltrimethylammoniumionen, sind ideal in der Lage, die positiv geladene Gruppe in einen ausreichend kleinen Abstand an das negativ geladene Sauerstoffatom der Nukleinsäure anzunähern, sodass eine ausreichende (lokale) Abschirmung der Gesamtladung beider Ionen bewirkt wird. Dabei bilden sich vorzugsweise Ionenpaare zwischen dem geladenen Sauerstoff der Phosphatgruppen der Nukleinsäure und den Ammoniumgruppen der quartären Amine.

Vorteilhaft an der Einstellung eines solchen Verhältnisses ist, dass der Komplex aus organischen Kationen und Nukleinsäure insgesamt annähernd ladungsneutral ist, d.h. im Wesentlichen vollständig komplexiert ist. Die komplexierte Nukleinsäure ist daher in einem wässrigen Medium unlöslich und kann bei der Herstellung in wässrigen Medien als unlösliches Präzipitat abgetrennt werden. Diese Eigenschaft erleichtert somit die Herstellung der gewünschten, stöchiometrisch komplexierten Nukleinsäure. Das Abtrennen der Komplexe gemäß Schritt iii) erfolgt daher vorzugsweise mittels Zentrifugation oder Filtration und ggf. nachgeschalteten Wasch- und Trocknungsschritten. Diese Verfahren stellen eine besonders einfache und effiziente Art der Abtrennung der präzipitierten Komplexe dar. Das Präzipitat kann z.B. durch Zentrifugation zum Beispiel für 1 bis 10 Minuten, vorzugsweise 5 Minuten, bei 10000 bis 20000 g, vorzugsweise 15000 g, abgetrennt werden. Gegebenenfalls kann dieser Vorgang nach erneuter Aufnahme des gefällten Niederschlags in Wasser mehrmals durchgeführt werden. Vorteilhaft an der Einstellung eines solchen Verhältnisses ist ferner, dass beim In-Kontakt-Bringen der so komplexierten Nukleinsäure mit einer lebenden Zelle oder einem lebenden Zellgewebe (z.B. der Haut) keine bzw. nur wenige freie organische Kationen mit den Zellen oder dem Zellgewebe wechselwirken können. Als Folge davon können die organischen Kationen kaum cytotoxischen Effekte oder andere Störungen des Metabolismus bei den Zellen auslösen, was ansonsten häufig vorkommen kann.

Im Zuge der Komplexierung der mindestes einen Nukleinsäuren können selbstverständlich wie oben erwähnt auch verschiedene organische Kationen Verwendung finden, indem man Mischungen unterschiedlicher organischer Kationen zu der Nukleinsäure-haltigen wässrigen Flüssigkeit gibt, um die Nukleinsäure-Komplexe zu präzipitieren.

Die durch Präzipitation erhaltenen Komplexe können, vorzugsweise nach Trocknung, anschließend in einer großen Vielzahl von organischen Lösungsmitteln (einschließlich solchen, die allgemein als Fällungsmittel für Nukleinsäure aus wässriger Lösung gelten) gelöst werden. Nach Aufnahme in ein Lösungsmittel enthalten diese im Wesentlichen vorzugsweise keine organischen Kationen, die über die zur Kompensation der negativen Ladungen der Nukleinsäure erforderliche Menge hinaus gehen. Dies bedeutet, dass die Anzahl der gesamten positiven Ladungen der organischen Kationen in dem Lösungsmittel im Wesentlichen mit der Anzahl der negativen Ladungen der Nukleinsäure identisch ist.

Organische Lösungsmittel, die zur Aufnahme der komplexierten Nukleinsäure geeignet sind, umfassen insbesondere ein- oder mehrwertige Alkohole oder teilweise veretherte Alkohole. Bevorzugt enthält das Lösungsmittel daher mindestens eine Ether-Gruppe und/oder mindestens eine Hydroxyl-Gruppe. Als Lösungsmittel besonders gut geeignete (amphiphile) Verbindungen können durch die Formel HO-R1-O-R2 beschrieben werden. Dabei ist R1 und R2 jeweils ein Kohlenwasserstoffrest mit 1 bis 100 Kohlenstoffatomen. Insbesondere sind C1 bis C5-Alkohole geeignet, wie z.B. Methanol, Ethanol, 1-Propanol oder 2-Propanol, Butanol, wie 1-Butanol, oder Pentadiol, wie 1-Pentadiol. Ferner sind mehrwertige Alkohole, wie Ethylenglykol oder teilweise veretherte Derivate davon, wie z.B. Ethylenglykol, das mit Methanol, Ethanol, Propanol oder Butanol verethert ist, wie beispielsweise Ethylenglykolether, wie insbesondere Ethylenglykolmonobutylether, -ethylether oder methylether, verwendbar. Auch Propylenglykol, Glycerin und Polyethylenglykole (PEG) können verwendet werden, insbesondere als Mischungen mit primären Alkoholen. DMSO gehört zu den bevorzugten Lösungsmitteln, da DMSO komplexierte Nukleinsäuren gut, insbesondere mit quartären Aminen komplexierte RNA zu Konzentrationen größer als 1mg/ml löst und darüber gut über die Haut aufgenommen wird. DMSO gemischt mit Propylenglykol ist eine besonders bevorzugte Lösungsmittelkombination. Weitere organische Lösungsmittel, die auch in Frage kommen, insbesondere bei der Verwendung von Lösungsmittelgemischen, sind Öle, Wachse und Fette.

Insbesondere bei Verwendung von niederen Alkoholen wie Ethanol, Propanol oder Butanol als Lösungsmittel lassen sich Konzentrationen an komplexierter Nukleinsäure erreichen, die ansonsten nur in neutralen oder wässrigen Medien erreicht werden. So konnten bei Lösung von Heringssperma-DNA, die in einem ladungs-stöchiometrischen Verhältnis von etwa 1:1 mit CTAB komplexiert war, in den Lösungsmitteln Methanol, Ethanol, 1-Butanol, 2-Propanol, 1-Pentanol und Ethylenglykolmonobutylether jeweils eine Löslichkeit von mehr als 10 mg DNA pro ml Lösungsmittel erreicht werden.

In Lösungsmitteln, die alleine eine geringere Löslichkeit für die erfindungsgemäß komplexierten Nukleinsäuren zeigen, können ebenfalls Konzentration an gelöster Nukleinsäure von mehr als 0,1 mg/ml erreicht werden, indem die Nukleinsäure zunächst in einem ersten Lösungsmittel mit hoher Löslichkeit (wie z.B. Methanol, Ethanol, 1-Butanol, 2-Propanol, 1-Pentanol, Ethylenglykolmonobutylether oder Mischungen derselben) gelöst wird, und das die komplexierten Nukleinsäuren enthaltende erste Lösungsmittel anschließend mit einem zweiten Lösungsmittel homogen vermischt wird, das eine geringere Löslichkeit für die erfindungsgemäß komplexierten Nukleinsäuren aufweist. Geeignete Beispiele hierfür sind Öle, Wachse und Fette. In Walnussöl, Weizenkeimöl, Leinöl, Distelöl, Rapsöl und Mandelöl konnte so eine Konzentration von 100µg/ml erreicht werden.

In bevorzugten Ausführungsformen der vorliegenden Erfindung umfassen die erfindungsgemäßen Zusammensetzungen daher mindestens ein Öl und/oder mindestens ein Wachs und/oder mindestens ein Fett.

Geeignete Öle, die zur Herstellung der erfindungsgemäßen Zusammensetzungen verwendet werden können, umfassen synthetische, tierische und pflanzliche Öle. Ebenso umfassen geeignete Wachse bzw. Fette synthetische, tierische und/oder pflanzliche Wachse bzw. synthetische, tierische und/oder pflanzliche Fette.

Geeignete pflanzliche Öle sind z.B. Nussöle und Samenöle. Geeignete pflanzliche Öle umfassen insbesondere Erdnussöl, Walnussöl, Mandelöl, Haselnussöl, Kokosnussöl, Sojabohnenöl, Olivenöl, Mohnöl, Hanföl, Kürbiskernöl, Sonnenblumensamenöl, Sesamsamenöl, Baumwollsamenöl, Distelöl, Leinöl, Rapsöl, Ricinusöl und Ähnliche. Auch aus Getreide gewonnene Keimöle können vorliegend verwendet werden. Bevorzugte Keimöle umfassen z.B. solche, die aus Mais, Weizen, Hafer, Roggen, Reis und Triticale gewonnen werden. Als geeignete pflanzliche Fette können beispielsweise Kokosfett oder Kakaobutter verwendet werden. Mögliche pflanzliche Wachse umfassen bspw. Zuckerrohrwachs, Carnaubawachs, Jojobaöl, Candelillawachs und Japanwachs.

Ein erfindungsgemäß besonders bevorzugte Mischung von organischen Lösungsmitteln umfasst Ethanol und Rizinusöl.

Neben pflanzlichen Ölen, Wachsen und Fetten können auch tierische Fette, Wachse und Öle, wie z.B. Fischöle oder aus Nerzen oder Hirschen gewonnene Öle und Fette, eingesetzt werden. Lebertran und Walöl (wie beispielsweise aus Spermaceti) sind Beispiele für Fischöle, die vorliegend verwendet werden können. Mögliche Quellen für tierische Wachse sind beispielsweise Walrat, Wollwachs und Bienenwachs. Geeignete Verfahren zur Gewinnung reiner Öle tierischen Ursprungs sind im Stand der Technik bekannt.

Ggf. können auch synthetische Öle, Fette und Wachse eingesetzt werden. Geeignete synthetische Öle und Fette basieren zum Beispiel auf Silikon- oder Paraffinverbindungen. Ein Beispiel ist Vaseline. Sojawachs kann durch Hydrierung aus Soja gewonnen werden und ist ein Beispiel für ein synthetisches Wachs.

Wie bereits angedeutet umfassen die Zusammensetzungen der vorliegenden Erfindung wenn darin ein Öl, Wachs oder Fett vorliegt, bevorzugt mindestens ein weiteres Lösungsmittel, das kein Öl, Wachs oder Fett ist. Oder anders formuliert: besonders bevorzugt umfassen die erfindungsgemäß zu verwendenden Zusammensetzungen neben einem (konventionellen) organischen Lösungsmittel wie Ethanol, Ethylenglykolmonobutylether, Ethylenglykolmonoethylether, Ethylenglykolmonomethylether, und/oder DMSO etc. noch ein Öl, Fett oder Wachs. Eine besonders bevorzugte Kombination ist eine Kombination von Ethanol und/oder DMSO mit mindestens einem Öl, Fett und/oder Wachs.

Die erfindungsgemäßen Zusammensetzungen können zum Beispiel einen Öl-, Wachs und/oder Fettanteil aufweisen, der größer oder gleich 25% (v/v), beispielsweise größer oder gleich etwa 30% (v/v), größer oder gleich etwa 40% (v/v), größer oder gleich etwa 50% (v/v), größer oder gleich etwa 60% (v/v), größer oder gleich etwa 70% (v/v), größer oder gleich etwa 80% (v/v), oder größer oder gleich etwa 85% (v/v) ist. In besonders bevorzugten Ausführungsformen weisen die erfindungsgemäßen Zusammensetzungen einen Öl-, Wachs und/oder Fettanteil von mehr als etwa 90%, vorzugsweise etwa 91 % (v/v) oder mehr, etwa 92% (v/v) oder mehr, etwa 93% (v/v) oder mehr, etwa 94% (v/v) oder mehr, etwa 95% (v/v) oder mehr, etwa 96% (v/v) oder mehr, etwa 97% (v/v) oder mehr, etwa 98% (v/v) oder mehr, etwa 99% (v/v) oder mehr auf.

Für das erfindungsgemäße Verfahren sind die Nukleinsäurekomplexe besonders bevorzugt gelöst in einem organische Lösungsmittel ausgewählt aus der Gruppe bestehend aus:
a) Alkohol, wie Ethanol, 1-Butanol, 2-Propanol, 1-Pentanol,
b) DMSO, und
c) Glycolether wie z.B. Ethylenglykolmonobutylether.

Da wie bereits erwähnt die Nukleinsäurekomplexe auch in homogenen Mischungen von Lösungsmitteln gelöst vorliegen können, ist es ferner bevorzugt, wenn die Komplexe aus mindestens einer Nukleinsäure und mindestens einem organischen Kation in der erfindungsgemäß zu verwendenden Zusammensetzung in einem, vorzugsweise homogenen, Gemisch gelöst vorliegen ausgewählt aus der Gruppe bestehend aus:
a) zwei oder mehr Alkoholen,
b) einem oder mehr Alkoholen mit DMSO,
c) einem oder mehr Alkoholen mit einem oder mehr Glycolethern,
d) DMSO und einem oder mehr Glycolethern,
e) DMSO, einem oder mehr Alkoholen, und einem oder mehr Glycolethern,
f) einem Alkohol und einem Öl,
g) DMSO und einem Öl,
h) Glycolether und einem Öl,
i) Gemischen von, vorzugsweise homogenen, Lösungsmittelgemischen gemäß a) bis e) mit einem oder mehr Ölen, oder
j) Gemischen von, vorzugsweise homogenen, Lösungsmittelgemischen gemäß a) bis i) mit einer wässrigen Flüssigkeit.

Die Gemische nach f) bis i) sind erfindungsgemäß besonders bevorzugt, wenn die Zusammensetzung mit Haut in Kontakt gebracht werden soll, um Haarentfernung und/oder Prävention des Nachwachsens von Haaren; und/oder Depigmentierung und/oder Prävention der Repigmentierung der Haut hervorzurufen.

Die in organischen Lösungsmitteln gelösten Nukleinsäurekomplexe zeichnen sich durch eine außerordentlich hohe Stabilität aus. Dies ist im Wesentlichen darauf zurückzuführen, dass Nukleasen in nicht-wässriger Umgebung inaktiv sind. Dies ist vor allem bei Verwendung von Ribonukleinsäure (RNA) als Inhibitor der Tyrosinasegenexpression von Bedeutung, da in allen wässrigen Systemen mit einer hohen RNAse-Aktivität gerechnet werden muss. Außerhalb einer RNAse-freien Umgebung, die praktisch nur unter speziellen Laborbedingungen realisiert werden kann, ist grundsätzlich mit einem schnellen Abbau der RNA zu rechnen. Dieses Problem wird durch die Komplexierung der Nukleinsäure zu den vorliegend beschriebenen Komplexen gelöst.

Die erfindungsgemäße Zusammensetzung umfasst vorzugsweise nur einen geringen Wasseranteil. Vorzugsweise ist der Anteil an Wasser in der Zusammensetzung 50% (v/w) oder weniger, bevorzugt 10% (v/w) oder weniger, besonders bevorzugt 5% (v/w) oder weniger.

Ferner ist die Stabilität der komplexierten Nukleinsäuren auch dadurch erhöht, dass in dem eingesetzten organischen Lösungsmittel vorzugsweise keine saure oder alkalische Umgebung mehr vorliegt. Dies vermeidet eine etwaige saure oder alkalische Hydrolyse der Nukleinsäuren.

Wie bereits erwähnt kann nach Lösung der Nukleinsäuren in dem mindestens einen organischen Lösungsmittel das organische Lösungsmittel beispielsweise mit beliebigen weiteren Flüssigkeiten vermischt werden, z.B. mit einem weiteren der oben genannten organischen Lösungsmittel oder einer wässrigen Flüssigkeit. In den erfindungsgemäß zu verwendenden Zusammensetzungen kann das mindestens eine organische Lösungsmittel beispielsweise weniger als 70% (v/v), z.B. weniger als 50% (v/v), oder weniger als 20% (v/v) ausmachen. Das mindestens eine organische Lösungsmittel kann auch in einer Konzentration von mehr als 1 % (v/v), bevorzugt mehr als 5%(v/v), besonders bevorzugt mehr als 10% (v/v) vorliegen. So kann das organische Lösungsmittel beispielsweise in einem Bereich von 1-70% (v/v), 5-50% oder auch 10-20% (v/v) vorliegen. Die Konzentration des mindestens einen organischen Lösungsmittels in der Zusammensetzung sollte so gewählt werden, dass beim In-Kontakt-Bringen der Zusammensetzung mit der Haut kaum oder gar keine toxischen Auswirkungen des mindestens einen organischen Lösungsmittels auf die Haut zu befürchten sind. Idealerweise wird als organisches Lösungsmittel bzw. Lösungsmittelgemisch bereits ein hautverträgliches Lösungsmittel bzw. Lösungsmittelgemisch gewählt. Insbesondere Lösungsmittelgemische mit Ölen sind in diesem Zusammenhang vorteilhaft.

Zusammensetzungen, die DMSO als organisches Lösungsmittel umfassen, weisen vorzugsweise einen DMSO-Gehalt im Bereich von 1% bis 60% (v/v), bevorzugt im Bereich von 2% bis 50% (v/v), besonders bevorzugt im Bereich von 5% bis 40% (v/v) auf.

Die erfindungsgemäße Zusammensetzung kann eine pharmazeutische Zusammensetzung sein, die ferner ggf. einen pharmazeutisch verträglichen Hilfsstoff, Träger, oder Exzipienten umfasst.

In einem weiteren Aspekt betrifft die vorliegende Erfindung die Verwendung einer erfindungsgemäßen Zusammensetzung bei einem Tier oder Menschen zur:
i) Haarentfernung und/oder Prävention des Nachwachsens von Haaren; und/oder
ii) Depigmentierung und/oder Prävention der Repigmentierung der Haut.

Das Tier ist besonders bevorzugt ein Säugetier, insbesondere ein Säugetier ausgewählt aus der Gruppe bestehend aus: Affe, Maus, Ratte, Hamster, Meerschweinchen, Hund, Katze, Pferd, Rind, Kaninchen, Hase, Schwein, Schaf, Ziege und andere. Bevorzugt ist aber die Behandlung am Menschen.

Unter "Prävention des Nachwachsens von Haaren" ist hier die absolute Verhinderung des Nachwachsens von Haaren als auch die Verringerung der Geschwindigkeit des Nachwachsens von Haaren oder die Verringerung der Menge an nachwachsenden Haaren im Vergleich zum Normalzustand zu verstehen. Depigmentierung bedeutet zumindest eine Verringerung des Pigmentierungsgrads, umfasst aber auch die vollständigen Depigmentierung. Der Begriff "Prävention der Repigmentierung" umfasst die absolute Verhinderung des Neupigmentierung als auch die Verringerung des Pigmentierungsgrads oder Pigmentierungsrate im Vergleich zum Normalzustand. Die Zusammensetzungen der vorliegenden Erfindung können daher nicht nur zur Entfernung von Haaren bzw. Pigmentierungen verwendet werden, sondern können auch, z.B. wenn die Haare und Pigmente bereits auf anderem Wege entfernt wurden, dazu verwendet werden, dass das Nachwachsen der Haare bzw. die Repigmentierung ausbleibt oder zumindest verringert oder verzögert wird.

Diese Verwendung kann nicht-therapeutischen, nicht-chirurgischen Zwecken dienen, z.B. rein kosmetischen Zwecken ohne therapeutischen Grund oder chirurgischen Eingriff, sie kann aber auch therapeutischen Zwecken dienen und/oder begleitend zu einem chirurgischen Eingriff angewendet werden. Beispielsweise können die erfindungsgemäßen Zusammensetzungen zur Behandlung von Hypertrichose, Hirsutismus, und/oder Hyperpgimentierung verwendet werden.

Besonders bevorzugt ist die Verwendung einer anti-Tyr siRNA zur Haarentfernung und/ oder Prävention des Nachwachsens von Haaren; und/oder Depigmentierung und/oder Prävention der Repigmentierung der Haut.

Die vorliegende Erfindung betrifft auch ein Verfahren zur Haarentfernung und/ oder Prävention des Nachwachsens von Haaren; und/oder Depigmentierung und/oder Prävention der Repigmentierung der Haut, bei einem Tier oder Menschen, wobei das Verfahren folgenden Schritt umfasst:
i) Verabreichen einer erfindungsgemäßen Zusammensetzung an das Tier oder den Menschen.

Insbesondere umfasst das Verabreichen das In-Kontakt-Bringen einer erfindungsgemäßen Zusammensetzung mit der Haut des Tier oder Menschen.

Die Zusammensetzungen der vorliegenden Erfindung können im Prinzip in beliebiger Art und Weise verabreicht werden einschließlich, aber nicht darauf beschränkt, auf oralem, enteralem oder parenteralem Wege, einschließlich intravenöser, intraarterieller, intramuskulärer, intraperitonealer, subkutaner, transdermaler, intratekaler, nasaler, rektaler, vaginaler und topischer Verabreichung (einschließlich buccaler und sublingualer Verabreichung). Die Verabreichung als Aerosol oder epidural ist theoretisch auch denkbar. Der bevorzugte Verabreichungsweg der erfindungsgemäßen Zusammensetzungen ist jedoch die topische Verabreichung direkt auf die Haut bzw. die behaarte und/oder pigmentierte Stelle. Daneben ist die subkutane, intrakutane, subdermale und dermale Verabreichung im Bereich oder zumindest in der Nähe des zu behandelnden Hautareals bevorzugt. Ein besonders bevorzugte Ausführungsform umfasst ein Hautpflaster, dass die erfindungsgemäßen Zusammensetzung an das zu behandelnde Hautareal abgibt.

Das Tier ist wie bereits erwähnt besonders bevorzugt ein Säugetier, insbesondere ein Säugetier ausgewählt aus der Gruppe bestehend aus: Affe, Maus, Ratte, Hamster, Meerschweinchen, Hund, Katze, Pferd, Rind, Kaninchen, Hase, Schwein, Schaf, Ziege und andere. Bevorzugt ist aber die Behandlung am Menschen.

Diese Verfahren können nicht-therapeutischen, nicht-chirurgischen Zwecken dienen, z.B. rein kosmetischen Zwecken ohne therapeutischen Grund oder chirurgischen Eingriff, sie können aber auch therapeutischen Zwecken dienen und/oder begleitend zu einem chirurgischen Eingriff angewendet werden. Beispielsweise können die erfindungsgemäßen Verfahren der Behandlung von Hypertrichose, Hirsutismus, und/oder Hyperpgimentierung dienen.

Das In-Kontakt-Bringen kann eine längere Inkubation umfassen. Üblicherweise wird die Exposition im Bereich von Minuten oder Stunden liegen. Bei einigen Anwendungen kann es allerdings auch vorteilhaft sein, eine Verweildauer von mehreren Tagen vorzusehen, z.B. wenn ein Pflaster wie hierin beschrieben verwendet wird. Die Inkubation kann daher über mehrere Minuten, z.B. mehr als 15 Minuten, erfolgen, oder auch für Stunden, zum Beispiel über Nacht, oder gar über Tage hinweg.

Kurze Beschreibung der Figuren
- Fig. 1: zeigt den Längsschnitt eines Haars aus einem Hautabschnitt einer Maus nach Behandlung mit fluoreszierenden Oligonukleotiden. Dargestellt sind die Zellkerne durch Färbung mit Bisbenzimid (Figur 1A) als auch die grüne Fluoreszenz (helle Bereiche) des aufgetragenen FAM-Oligonukleotids an (Figur 1 B).
- Fig. 2: zeigt den Querschnitt eines Haars aus einem Hautabschnitt einer Maus nach Behandlung mit fluoreszierenden Oligonukleotiden. Dargestellt sind die Zellkerne durch Färbung mit Bisbenzimid (Figur 2A) als auch die grüne Fluoreszenz (helle Bereiche) des aufgetragenen FAM-Oligonukleotids an (Figur 2B).

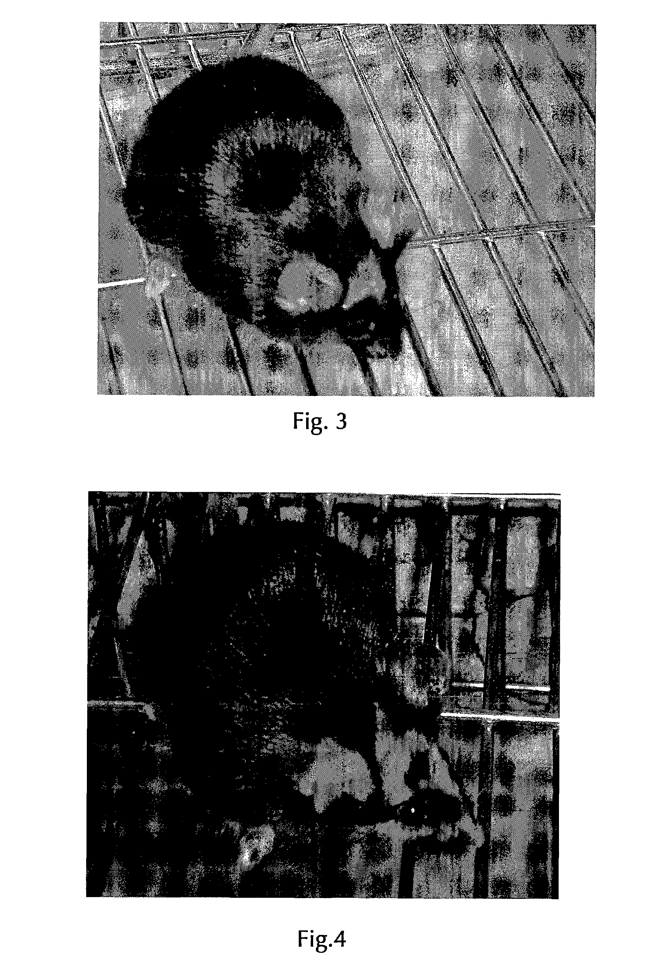

- Fig. 3: Fotografie einer Maus vier Wochen nach Behandlung mit siRNA-1 (Tyrosinase) (SEQ ID NO: 4) in DMSO-Propylenglycol-Formulierung.
- Fig. 4: Fotografie einer Maus vier Wochen nach Behandlung mit siRNA-1 (Tyrosinase) (SEQ ID NO: 4) in DMSO-Propylenglycol-Formulierung.

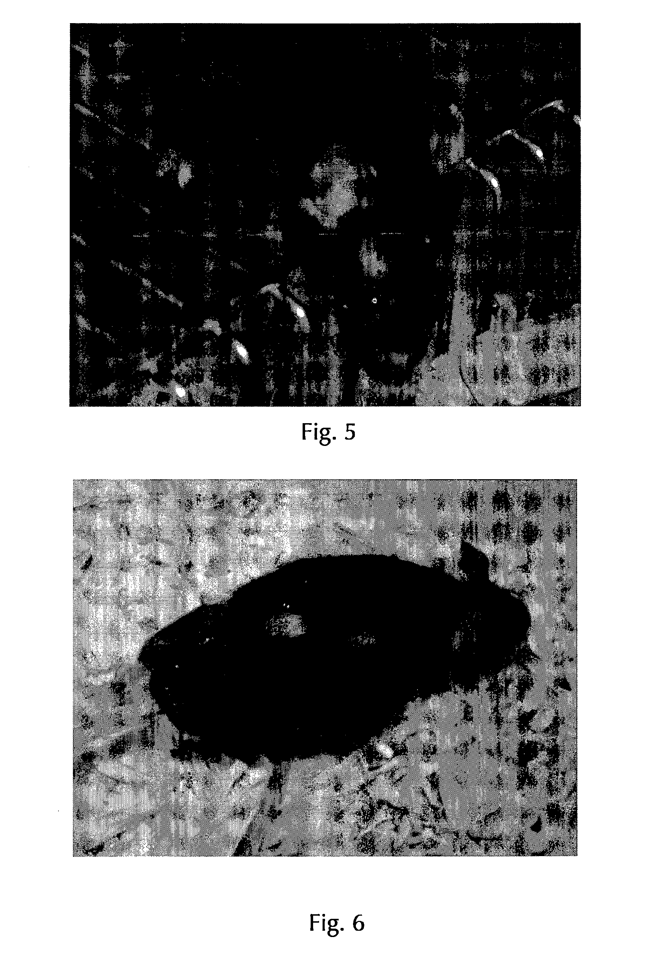

- Fig. 5: Fotografie einer Maus drei Wochen nach Behandlung mit siRNA-1 (Tyrosinase) (SEQ ID NO: 4) in DMSO-Ethanol-Rizinus-Formulierung.
- Fig. 6: Fotografie einer Maus drei Wochen nach Behandlung mit siRNA-1 (Tyrosinase) (SEQ ID NO: 4) in DMSO-Ethanol-Rizinus-Formulierung.

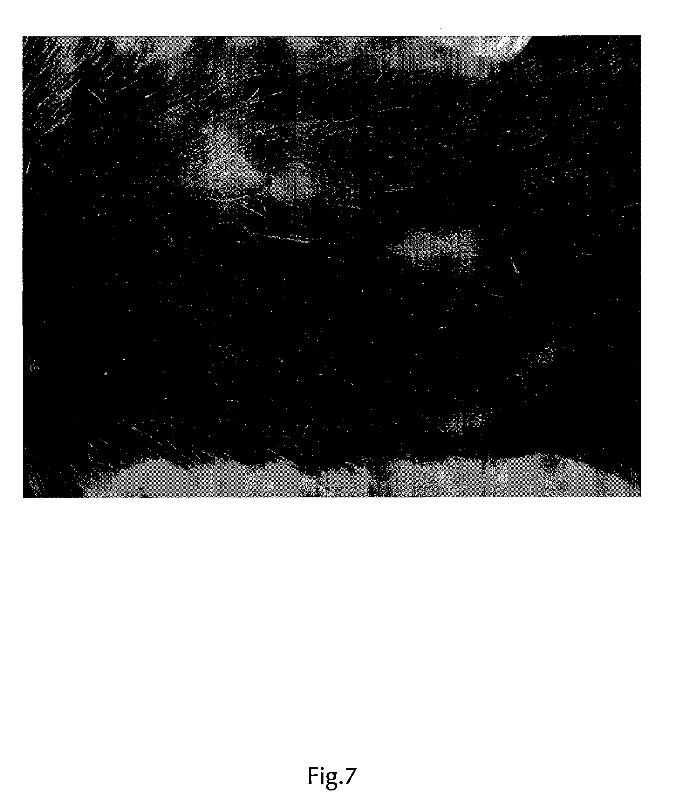

- Fig. 7: Fotografie einer Maus fünf Wochen nach Behandlung mit siRNA-1 (Tyrosinase) (SEQ ID NO: 4) in DMSO-Ethanol-Rizinus-Formulierung.

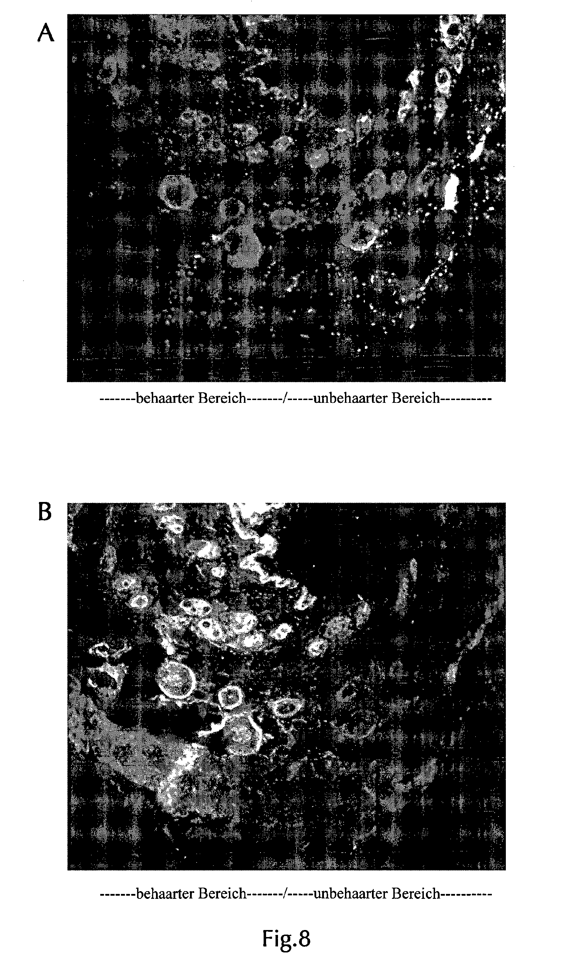

- Fig. 8: Fluoreszenz-mikroskopische Aufnahmen eines Dünnschnitts eines Hautbereichs fünf Wochen nach Behandlung mit siRNA-1 (SEQ ID NO: 4) in Dimethylsulfoxid/Propylenglycol. Es ist ein Hautbereich präpariert, der den Übergang zwischen der haarlosen und dem behaarten Bereich zeigt. Fig. 8A: Darstellung der Zellkerne mit Bisbenzimid. Fig. 8B: Darstellung von Tyrosinase mittels anti-Tyrosinase-Antikörpern.

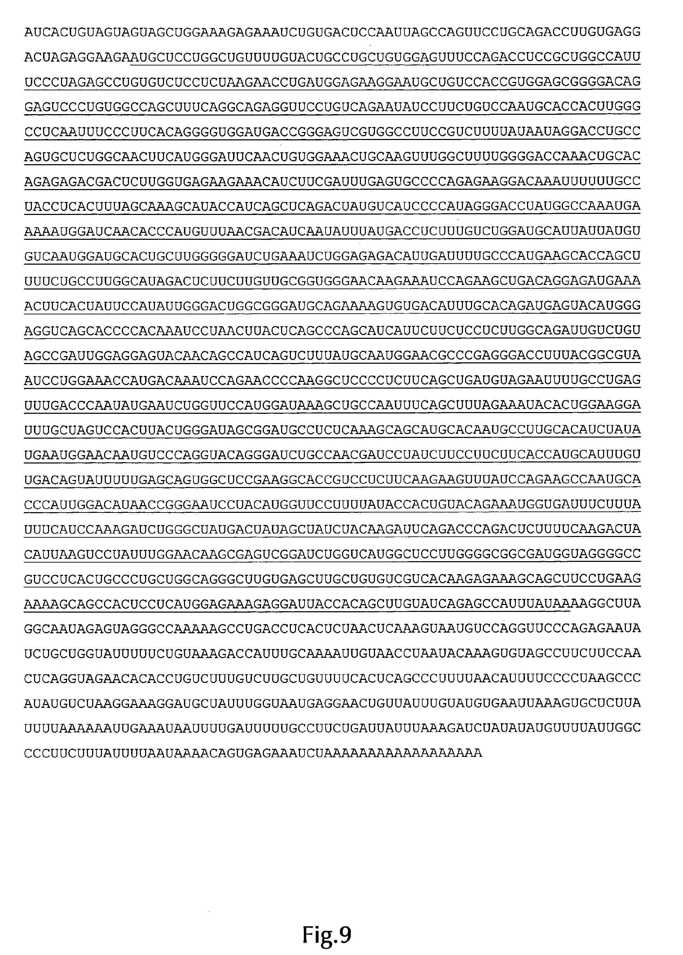

- Fig. 9: Sequenz der humanen Tyrosinase mRNA (SEQ ID NO: 1). Die unterstrichene Sequenz stellt die kodierende Sequenz der humanen Tyrosinase dar (SEQ ID NO: 2).

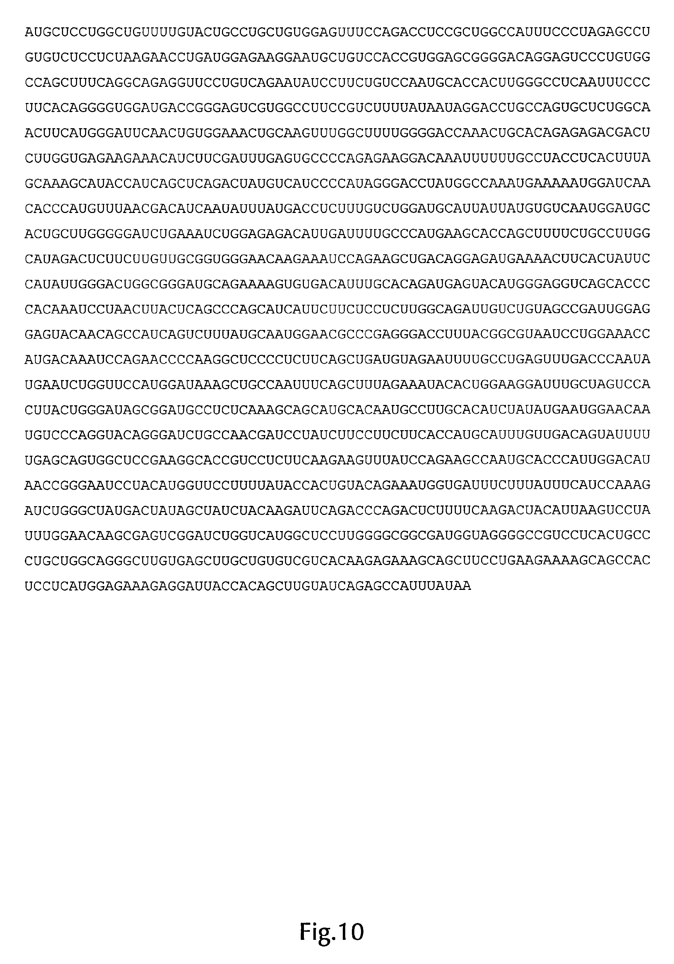

- Fig. 10: Kodierende RNA Sequenz der humanen Tyrosinase (SEQ ID NO: 2).

Die folgenden Ausführungsbeispiele veranschaulichen weitere bevorzugte Ausführungsformen der vorliegenden Erfindung, wobei die vorliegende Erfindung nicht auf diese spezifischen Ausführungsbeispiele beschränkt ist.

### Beispiel 1

### Herstellung von Oligonukleotiden

Folgende Oligonukleotide wurden für die transdermale Verabreichung an menschliche Haut bzw. Mäusehaut vorgesehen.

Ein Oligonukleotid (3'-GAT CCT GCA TAT GGT AGT G -5' (SEQ ID NO: 3), Molekulargewicht 5938,82g/Mol) war mit dem fluoreszenten Farbstoff TAMRA markiert (Oligonukleotid 1). Oligonukleotid 2 (Molekulargewicht 5938,82g/Mol) mit der selben Sequenz war mit dem fluoreszenten Farbstoff Fluorescein 6FAM markiert, um einen Nachweis des Oligonukleotids in den Hautzellen zu ermöglichen.

Die Oligonukleotide wurden von der Firma Biolegio (6545 CG Nijmegen, Niederlande) bezogen.

### Beispiel 2

Zur Herstellung von CTAB-Oligonukleotid-Komplexen wurde 1 mg der Oligonukleotide in 200µl Wasser gelöst und mit 400µl 10mM CTAB versetzt. Die Probe wurde 30min auf Eis inkubiert und der entstandene Niederschlag 5min bei 10000 xg zentrifugiert. Der Überstand wurde abgezogen und der Niederschlag mit 200 µl Wasser suspendiert und erneut zentrifugiert. Der Niederschlag wurde über Nacht im Speedvac getrocknet.

### Beispiel 3

### Herstellung von lipophiler Nukleinsäure-Komplexe in Rizinusöl

5mg des fluoreszierenden DNA-Oligonukleotid 2 wurde in 1ml Wasser gelöst und mit 300µl 100mM Cetyltrimethylammoniumbromid-Lösung gemischt. Es entstand eine gelbliche Trübung, die durch 5min Zentrifugation bei 10.000 xg niedergeschlagen wurde. Der Überstand wurde verworfen und der Niederschlag in 1ml Wasser suspendiert und erneut wie oben zentrifugiert. Der Überstand wurde verworfen und der Niederschlag unter Vakuum getrocknet. Der Niederschlag wurde in 1ml Ethanol aufgenommen. Es entstand eine gelbe, klare Lösung. 100µl der fluoreszierenden DNA wurden in einem Kunststoffröhrchen mit 1ml Rizinusöl gemischt. Es entstand eine homogene, zähe Flüssigkeit, die 90% (v/v) Rinizinusöl und 10% (v/v) Ethanol und gelöste DNA enthielt.

### Beispiel 4

### Einbringung von fluoreszierender DNA in Haarzellen durch Applikation der lipophilen Nukleinsäurekomplexen auf die Haut von Mäusen

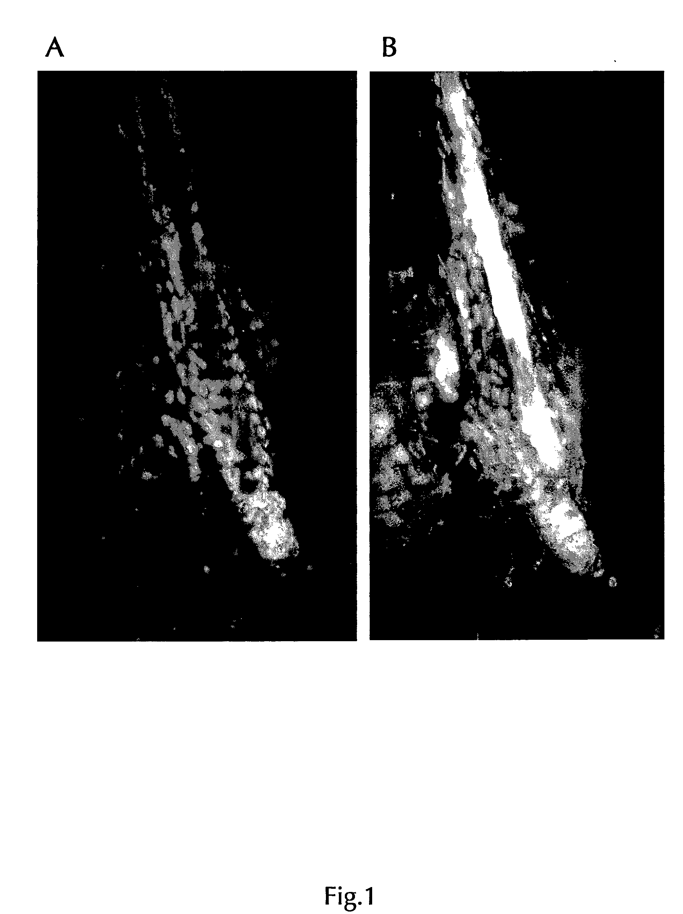

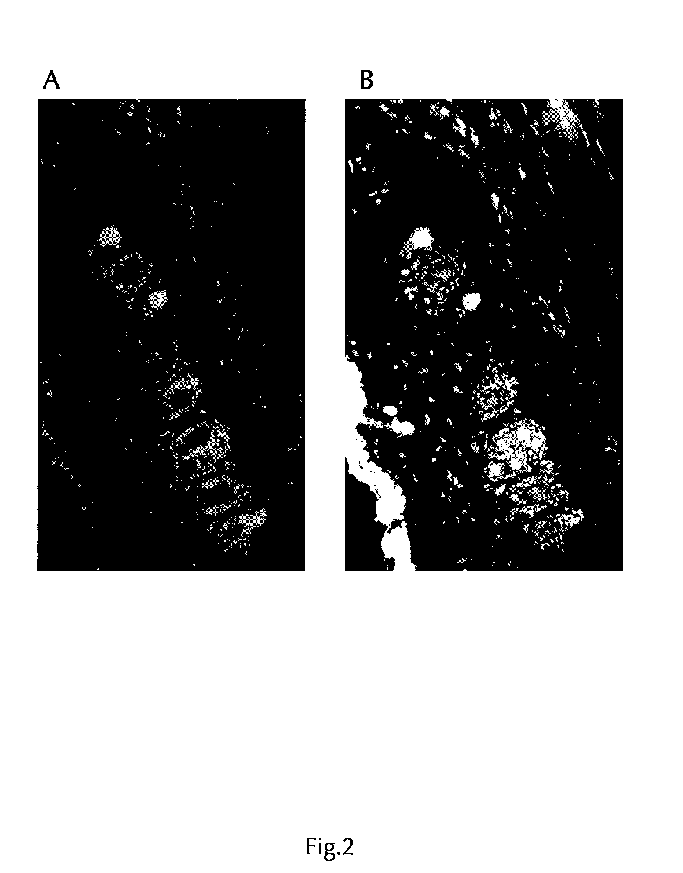

3 Mäuse (C27/Black) wurden für die dermale Applikation der Zusammensetzung durch Rasur eines Fellbereichs im Nacken vorbereitet. Je einer Maus wurden jeweils 10µl, 20µl und 40 µl der Zusammensetzung nach Beispiel 3 auf den rasierten Nackenbereich aufgetragen. Nach drei Tagen wurden die Mäuse getötet und die behandelten Hautbereiche herausgeschnitten. Als Kontrolle wurden Hautbereiche verwendet, die nicht mit Nukleinsäurekomplexen behandelt waren. Die Präparate wurden durch Inkubation über Nacht bei 4°C in 4% (w/v) Formaldehyd in mit Phosphat gepufferter Saline (PBS) fixiert und in flüssigen Stickstoff eingefroren. Aus den Hautbereichen wurden Dünnschnitte einer Dicke von etwa 8 µm auf Objektträgern für die Fluoreszenzmikroskopie hergestellt. Eine Kernfärbung erfolgte durch 5min Inkubation in 2µg/ml Bisbenzimid in PBS. Die Dünnschnitte wurden mit Fluoromount beschichtet und mittels Fluoreszenzmikroskop untersucht. Die Dünnschnitte, der mit fluoreszierenden DNA-Oligonukleotid 2 behandelten Bereiche zeigten eine grüne Fluoreszenz innerhalb der Haut, die im Besonderen in den Zellkernen nachweisbar war. In Dünnschnitten von nicht behandelten Hautbereichen war keine Fluoreszenz zu beobachten. Die Figuren 1 und 2 zeigen im Längs- (Figur 1) und Querschnitt (Figur 2) Haare, wobei die Zellkerne von Haarzellen durch das Fluoreszein-markierte Oligonukleotid 2 grün gefärbt waren. Haarzellen aus Bereichen, die nicht mit fluoreszierender DNA behandelten waren, zeigten keinerlei grüne Fluoreszenz innerhalb der Zellen.

### Beispiel 5

### Herstellung von Oligonukleotiden zur Beeinflussung des Haarwachstums und Pigmentbildung der Haut

siRNAs wurden als doppelsträngige Produkte mit 3' Desoxythymidinüberhängen (dTdT) von Eurofins MWG Operon (85560 Ebersberg, Deutschland) bezogen.

siRNA-1 (Tyrosinase-spezifisch) (5'-CUA UCU GAA UCA GAU UCU A dTdT-3'; SEQ ID NO: 4), mit zwei Desoxythymidinen am 3'-Ende und entsprechend komplementären RNA-Gegenstrang ebenfalls mit zwei Desoxythymidinen am 3'-Ende.

siRNA-2 (unspezifische Kontrolle) (5'-GCU GAC CCU GAA GUU CAU C dTdT-3'; SEQ ID NO: 5), mit zwei Desoxythymidinen am 3'-Ende und entsprechend komplementären RNA-Gegenstrang ebenfalls mit zwei Desoxythymidinen am 3'-Ende.

### Beispiel 6

### Herstellung von komplexierter siRNA in Ethanol/Rizinusöl

2mg des siRNA-Oligonukleotids 1 (SEQ ID NO: 4) (Tyrosinase-spezifisch) und des siRNA-Oligonukleotids 2 (unspezifische Kontrolle; SEQ ID NO: 5) wurden jeweils in 200µl Wasser gelöst und mit 600µl 10mM Cetyltrimethylammoniumbromid-Lösung gemischt. Es entstanden eine weiße Trübungen, die durch 5min Zentrifugation bei 10.000 xg niedergeschlagen wurden. Die Überstände wurden verworfen und die Niederschläge in je 200µl Wasser suspendiert und erneut wie oben zentrifugiert. Die Überstände wurden verworfen und die Niederschläge unter Vakuum getrocknet. Zu den Niederschlägen wurde zunächst 200µl Ethanol gegeben und die Suspension gevortext. Die Suspension blieb trübe. In 200µl Schritten wurde weiterer Ethanol zugefügt und gemischt. Bei der Zugabe von insgesamt 1200µl Ethanol war die komplexierte siRNA komplett gelöst. 50µl, 100µl, 200µl der komplexierten siRNA in Ethanol wurden mit 950µl, 900µl und 800µl Rizinusöl (Gesamtvolumen jeweils 1ml) durch Auf- und Abpipettieren gemischt. Es entstanden klare Lösungen mit siRNAs.

| Probe | siRNA-Sequenz | Spezifität | Konz. | Lösungsmittel %(v/v) | |
|---|---|---|---|---|---|
| | | | µg/ml | Ethanol | Rizinusöl |
| 1a | CUA UCU GAA UCA GAU UCU A dTdT (SEQ ID NO: 4) | Tyrosinase | 83 | 5 | 95 |
| 1b | CUA UCU GAA UCA GAU UCU A dTdT (SEQ ID NO: 4) | Tyrosinase | 167 | 10 | 90 |
| 1c | CUA UCU GAA UCA GAU UCU A dTdT (SEQ ID NO: 4) | Tyrosinase | 333 | 20 | 80 |
| 2a | GCU GAC CCU GAA GUU CAU C dTdT (SEQ ID NO: 5) | Kontrolle | 83 | 5 | 95 |
| 2b | GCU GAC CCU GAA GUU CAU C dTdT (SEQ ID NO: 5) | Kontrolle | 167 | 10 | 90 |
| 2c | GCU GAC CCU GAA GUU CAU C dTdT (SEQ ID NO: 5) | Kontrolle | 333 | 20 | 80 |

### Beispiel 7

### Herstellung von komplexierter siRNA in Dimethylsulfoxid/Propylenglycol

2mg der siRNA-Oligonukleotid 1 (SEQ ID NO: 4) und 2 (SEQ ID NO: 5) wurden jeweils in 200µl Wasser gelöst und mit 600µl 10mM Cetylammoniumbromid-Lösung gemischt. Es entstand eine weisse Trübung, die durch 5min Zentrifugation bei 10.000 xg niedergeschlagen wurde. Der Überstand wurde verworfen und der Niederschlag in 200µl Wasser suspendiert und erneut wie oben zentrifugiert. Der Überstand wurde verworfen und der Niederschlag unter Vakuum getrocknet. Der Niederschlag (Cetylammonium-siRNA) wurde in 200µl Dimethylsulfoxid aufgenommen. 10µl, 20µl und 50µl der komplexierten siRNA in Dimethylsulfoxid wurde mit 90µl, 80µl und 50µl Propylenglykol (Gesamtvolumen jeweils 100µl) gemischt. Es entstanden klare Lösungen mit gelösten siRNAs .

| Probe | siRNA-Sequenz | Spezifität | Konz. | Lösungsmittel %(v/v) | |
|---|---|---|---|---|---|
| | | | µg/µl | DMSO | Propylenglycol |
| 1d | CUA UCU GAA UCA GAU UCU A dTdT (SEQ ID NO: 4) | Tyrosinase | 1 | 10 | 90 |
| 1f | CUA UCU GAA UCA GAU UCU A dTdT (SEQ ID NO: 4) | Tyrosinase | 2 | 20 | 80 |
| 1g | CUA UCU GAA UCA GAU UCU A dTdT (SEQ ID NO: 4) | Tyrosinase | 4 | 40 | 60 |
| 2d | GCU GAC CCU GAA GUU CAU C dTdT (SEQ ID NO: 5) | Kontrolle | 1 | 10 | 90 |
| 2f | GCU GAC CCU GAA GUU CAU C dTdT (SEQ ID NO: 5) | Kontrolle | 2 | 20 | 80 |
| 2g | GCU GAC CCU GAA GUU CAU C dTdT (SEQ ID NO: 5) | Kontrolle | 4 | 40 | 60 |

### Beispiel 8

### Effekt von dermaler Applikation lipophilen siRNA-1 (Tyrosinase-spezifischen)-Komplexen in Mäusen

14 Mäuse wurden zur Untersuchung des Effekts von lipophilen siRNA-Tyr-Komplexen verwendet. 3 Mäusen wurden auf die unrasierte Kopf- bzw. Rückenhaut je 20µl der in Dimethylsulfoxid/Propylenglycol gelösten siRNA-1 (Proben 1d, 1f und 1g aus Beispiel 7) aufgetragen. 3 weiteren Mäusen wurden auf die unrasierte Kopf- bzw. Rückenhaut je 20µl der in Ethanol/Rizinusöl gelösten siRNA-1 (Proben 1a, 1b und 1c des Beispiel 6) aufgetragen. 3 weiteren Mäusen wurden auf die gleichen Stellen je 20µl der in Ethanol/Rizinusöl gelösten siRNA 2 (unspezifische Kontrollen, Proben 2a, 2b und 2c des Beispiel 6) und 3 weiteren Mäusen die in Dimethylsulfoxid/Propylenglycol gelöste siRNA 2 (unspezifische Kontrolle, Proben 2d, 2f und 2g des Beispiel 7) aufgetragen. Als weitere Kontrollen wurde unkomplexierte siRNA-1 unmittelbar in sterilem TE (10mM TrisCl, pH8, 2mM EDTA) bzw. TE mit 20% (v/v) Dimethylsulfoxid zu einer Endkonzentration an siRNA-1 von 2µg/µl gelöst und je 20µl dieser Lösung ebenfalls auf jeweils eine Maus aufgebracht. Danach wurden die Mäuse mit Wasser und Trockenfutter ad libitum gehalten. Nach ca. 2 Wochen färbten sich die Haare der Mäuse, die mit siRNA-1 (Tyrosinase-spezifisch) behandelt waren, weiß und fielen aus. Die Haare der zur Kontrolle mit siRNA-2 wie auch die mit unkomplexierter siRNA-1 behandelten Mäuse blieben unverändert dicht und schwarz. Nach weiteren 3 Wochen wuchsen die Haare, der mit siRNA-1 (Tyrosinase-spezifisch) wieder nach, so dass der Effekt nach ca. 7 Wochen nach Applikation vollkommen aufgehoben war. Abgesehen von der Haarveränderung war keine Abweichung der mit siRNA-1 (Tyrosinase-spezifisch) behandelten Mäuse im Vergleich zur Kontrollgruppe beobachtbar. Über den gesamten Zeitraum waren die Mäuse vital und zeigten keinerlei pathologische Veränderungen.

### Beispiel 9

### Nachweis der Reduktion der Melaninproduktion und Tyrosinaseexpression in Dünnschnitten siRNA-1 behandelter Hautbereiche

Zum Nachweis der spezifischen Reaktion der Tyrosinase-spezifischen siRNA-1 wurde das Tyrosinase-Protein in der Haut der behandelten Mäuse in Dünnschnitten mittels Anti-Tyrosinase-Antikörpern untersucht. Dazu wurde eine Maus, die mit komplexierter siRNA-1 in Dimethylsulfoxid/Propylenglycol (20%:80%) behandelt worden war, nach 5 Wochen getötet und ein Hautbereich heraus geschnitten, der sowohl einen Teil eines haarlosen wie auch einen Teil behaarten Hautbereich einschloss. Als Kontrolle wurde ein Hautbereich verwendet, der nicht mit komplexierter siRNA-1 behandelt worden war. Die Hautfragmente wurden über Nacht bei 4°C in PBS mit 4% Formaldehyd fixiert, in Flüssigstickstoff eingefroren und in 8 µm Dünnschnitte geschnitten. Die Schnitte wurden in 1 zu 200 in PBS verdünnten Kaninchen anti-Tyrosinase-Antikörpern (Antibody-online.com, Atlanta, GA 30346, USA) 1 Std. bei Raumtemperatur inkubiert. Die Schnitte wurden in PBS gespült und mit Rhodamin-gekoppelten anti-Kaninchen-Antikörpern 1 Stunde bei Raumtemperatur inkubiert. Darauf wurden die Schnitte mit PBS gespült und zur Kernfärbung mit 2µg/ml Bisbenzimid in PBS inkubiert. Darauf wurden die Schnitte wieder mit PBS gespült und mit einem Tropfen Fluoromount eingedeckelt. Die Dünnschnitte wurden mittels Fluorezenzmikroskop ausgewertet. Es zeigte sich in den Kontrollen, die nicht mit komplexierter siRNA-1 behandelt worden waren eine deutliche rote Fluoreszenz in Bereich der Haarzellen und Epithelzellen. Die rote Fluoreszenz zeigt das Vorhandensein von Tyrosinase in den Dünnschnitten an. Hautbereiche, die mit komplexierter siRNA-1 behandelt waren, zeigten keine bzw. stark reduzierte rote Fluoreszenz.

Der Einfluss der siRNA-Behandlung auf die Melaninproduktion wurde in Dünnschnitten mittels Durchlichtmikroskopie beobachtet. In unbehandelten Hautbereichen oder in Hautbereichen, die mit unspezifischer Kontroll-siRNA behandelt worden war, konnte Melanin - vor allem im Bereich um die Haarzellen - gut beobachtet werden, während in mit Tyrosinase-spezifischer siRNA-1 behandelten Hautbereichen keine oder im Vergleich zu den Kontrollen stark reduzierte Mengen an Melanin aufzufinden waren.

## Patentansprüche

1. Zusammensetzung umfassend einen Inhibitor der Tyrosinasegenexpression, wobei der Inhibitor der Tyrosinasegenexpression eine Nukleinsäure ist, die in der Lage ist, die Genexpression des Tyr Gens zu inhibieren, oder ein Inhibitor des Microphtalmia-assoziierten Transskriptionsfaktors (MITF) ist.

2. Die Zusammensetzung nach Anspruch 1, wobei der Inhibitor der Tyrosinasegenexpression eine Nukleinsäure ist, insbesondere wobei die Nukleinsäure eine Antisense RNA, eine siRNA oder micro RNA ist.

3. Die Zusammensetzung gemäß Anspruch 2, wobei die Nukleinsäure in der Lage ist, die humanen Tyrosinasegenexpression zu inhibieren.

4. Die Zusammensetzung gemäß Anspruch 2 oder 3, wobei die Nukleinsäure einen Sequenzabschnitt einer Länge von 19 bis 23 Nukleotiden, der mindestens 70% Sequenzidentität mit einem Sequenzabschnitt der kodierenden Sequenz der humanen Tyrosinase mRNA (SEQ ID NO: 2) oder einer dazu komplementären Sequenz aufweist, umfasst.

5. Die Zusammensetzung gemäß einem der Ansprüche 2 bis 4, wobei die Nukleinsäure eine siRNA ist, die die in der Lage ist, die Genexpression des humanen Tyrosinase Gens (Tyr Gen) zu inhibieren, und die folgendes umfasst:
i) einen Sequenzabschnitt einer Länge von 19 bis 23 Nukleotiden, der mindestens 70% Sequenzidentität mit einem Sequenzabschnitt der kodierenden Sequenz der humanen Tyrosinase mRNA (SEQ ID NO: 2) oder einer dazu komplementären Sequenz aufweist, und
ii) am 3'Ende eines oder beider Stränge einen einzelsträngigen Überhang von 1 bis 2 Nukleotiden aufweist, die vorzugsweise dT (Deoxythymidin) Nukleotide sind.

6. Die Zusammensetzung gemäß einem der Ansprüche 2 bis 5, wobei die Zusammensetzung mindestens ein organisches Lösungsmittel umfasst, und wobei die Nukleinsäure in Form von Komplexen mit mindestens einem organischen Kation in diesem mindestens einen organischen Lösungsmittel gelöst vorliegt.

7. Die Zusammensetzung gemäß Anspruch 6, wobei die Nukleinsäure in der Zusammensetzung derart komplexiert ist, dass die negativen Ladungen der Nukleinsäure durch organische Kationen im Wesentlichen vollständig kompensiert sind.

8. Die Zusammensetzung gemäß Anspruch 6 oder 7, wobei der Anteil an Wasser in der Zusammensetzung 50% (v/w) oder weniger ist, bevorzugt 10% (v/w) oder weniger ist, besonders bevorzugt 5% (v/w) oder weniger ist.

9. Die Zusammensetzung gemäß einem der Ansprüche 6 bis 8, wobei das mindestens eine organische Lösungsmittel ausgewählt ist aus der Gruppe bestehend aus:
a) ein Alkohol,
b) DMSO, und
c) Glycolether wie z.B. Ethylenglykolmonobutylether.

10. Die Zusammensetzung gemäß einem der Ansprüche 6 bis 9, wobei die Komplexe der Nukleinsäure und dem mindestens einem organischen Kation in der Zusammensetzung gelöst vorliegen in einem Gemisch von:
a) zwei oder mehr Alkoholen,
b) einem oder mehr Alkoholen mit DMSO,
c) einem oder mehr Alkoholen mit einem oder mehr Glycolethern,
d) DMSO und einem oder mehr Glycolethern,
e) DMSO, einem oder mehr Alkoholen, und einem oder mehr Glycolethern,
f) einem Alkohol und einem Öl,
g) DMSO und einem Öl,
h) einem Glycolether und einem Öl,
i) Gemischen von, vorzugsweise homogenen, Lösungsmittelgemischen gemäß a) bis e) mit einem oder mehr Ölen, oder
j) Gemischen von, vorzugsweise homogenen, Lösungsmittelgemischen gemäß a) bis i) mit einer wässrigen Flüssigkeit.

11. Die Zusammensetzung gemäß einem der Ansprüche 6 bis 10, wobei das mindestens eine organischen Kation eine Ammoniumverbindung ist, insbesondere wobei das organische Kation eine quartäre Ammoniumverbindung ist.

12. Die Zusammensetzung gemäß einem der Ansprüche 6 bis 11, wobei das organische Kation Cetyltrimethylammonium oder Benzalkonium ist.

13. Zusammensetzung gemäß einem der Ansprüche 6 bis 12, wobei die Zusammensetzung einen Öl-, Wachs oder Fettanteil von 30% oder mehr, vorzugsweise 50% oder mehr, besonders bevorzugt 80% oder mehr (v/v) besitzt.

14. Zusammensetzung gemäß einem der Ansprüche 6 bis 13, wobei der DMSO-Gehalt der Zusammensetzung im Bereich 1 % bis 60% (v/v), bevorzugt im Bereich 2% bis 50% (v/v), besonders bevorzugt im Bereich 5% bis 40% (v/v) liegt.

15. Zusammensetzung nach einem der vorhergehenden Ansprüche, wobei die Zusammensetzung eine pharmazeutische Zusammensetzung ist und ferner einen pharmazeutisch verträglichen Hilfsstoff, Träger, oder Exzipienten umfasst.

16. Verwendung einer Zusammensetzung gemäß einem der Ansprüche 1 bis 15 bei einem Tier oder Menschen zur nicht-therapeutischen, nicht-chirurgischen:
i) Haarentfernung und/ oder Prävention des Nachwachsens von Haaren; und/oder
ii) Depigmentierung und/oder Prävention der Repigmentierung der Haut.

17. Zusammensetzung zur Anwendung in einer chirurgischen und/oder therapeutischen Behandlung des menschlichen oder tierischen Körpers, wobei die Zusammensetzung eine Zusammensetzung gemäß einem der Ansprüche 1 bis 15 ist.

18. Zusammensetzung zur Anwendung nach Anspruch 17, wobei die Zusammensetzung zur Behandlung von Hypertrichose, Hirsutismus, und/oder Hyperpgimentierung angewendet wird.
